(19) Europäisches Patentamt
European Patent Office
Office européen des brevets

(11) **EP 4 545 180 A1**

(12) **EUROPÄISCHE PATENTANMELDUNG**

(43) Veröffentlichungstag:
**30.04.2025 Patentblatt 2025/18**

(21) Anmeldenummer: 24208349.1

(22) Anmeldetag: **23.10.2024**

(51) Internationale Patentklassifikation (IPC):
**B01L 3/00** *(2006.01)* **B01L 7/00** *(2006.01)*

(52) Gemeinsame Patentklassifikation (CPC):
**B01L 3/502738; B01L 7/52;** B01L 2300/0816;
B01L 2300/0864; B01L 2300/0867; B01L 2300/087;
B01L 2400/0672; B01L 2400/0677

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(30) Priorität: 24.10.2023 DE 102023129147

(71) Anmelder: **Technische Universität Dresden Körperschaft des öffentlichen Rechts 01069 Dresden (DE)**

(72) Erfinder:
• **RICHTER, Andreas 01219 Dresden (DE)**

• **GRUNER, Denise 01309 Dresden (DE)**
• **SHAHADHA, Mohammed 01099 Dresden (DE)**
• **BECK, Anthony 01219 Dresden (DE)**
• **OBST, Franziska 01237 Dresden (DE)**
• **FRIEDEMANN, Markus 01307 Dresden (DE)**
• **MENSCHIKOWSKI, Mario 01454 Radeberg (DE)**

(74) Vertreter: **Maikowski & Ninnemann Patentanwälte Partnerschaft mbB Postfach 15 09 20 10671 Berlin (DE)**

(54) **FLUIDISCHE VORRICHTUNG UND VERFAHREN ZUR PROBENVORBEREITUNG MIT EINER FLUIDISCHEN VORRICHTUNG**

(57) Die Erfindung betrifft eine fluidische Vorrichtung, aufweisend einen ersten Kanalabschnitt und n zweite Kanalabschnitte, wobei $n \in \mathbb{N}$ und $n \geq 1$, mindestens ein Öffnerelement und mindestens ein Schließerelement. Die erfindungsgemäße Vorrichtung ist dadurch gekennzeichnet, dass
• jeder der n zweiten Kanalabschnitte durch ein Öffnerelement angrenzend an den ersten Kanalabschnitt angeordnet ist;
• jedes Öffnerelement dazu eingerichtet ist, eine fluidische Verbindung von dem jeweiligen zweiten Kanalabschnitt zum ersten Kanalabschnitt herzustellen;
• der erste und die n zweiten Kanalabschnitte jeweils einen Aufnahmebereich aufweisen,
• im ersten Kanalabschnitt in Strömungsrichtung eines Fluids mindestens einem der Öffnerelemente ein Schließerelement vorgelagert angeordnet ist.

**Beschreibung**

Die Erfindung betrifft eine fluidische Vorrichtung, aufweisend einen ersten Kanalabschnitt und *n* zweite Kanalabschnitte, wobei $n \in \mathbb{N}$ und $n \geq 1$, mindestens ein Öffnerelement und mindestens ein Schließerelement. Die erfindungsgemäße Vorrichtung ist dadurch gekennzeichnet, dass

- jeder der *n* zweiten Kanalabschnitte durch ein Öffnerelement angrenzend an den ersten Kanalabschnitt angeordnet ist;
- jedes Öffnerelement dazu eingerichtet ist, eine fluidische Verbindung von dem jeweiligen zweiten Kanalabschnitt zum ersten Kanalabschnitt herzustellen;
- der erste und die *n* zweiten Kanalabschnitte jeweils einen Aufnahmebereich aufweisen,
- im ersten Kanalabschnitt in Strömungsrichtung eines Fluids mindestens einem der Öffnerelemente ein Schließerelement vorgelagert angeordnet ist.

Die Probenvorbereitung stellt sehr häufig einen zeitkritischen Schritt bei der schnellen und präzisen Diagnostik von z. B. Infektionserkrankungen dar und ist damit essentiell für die Einleitung einer erfolgreichen Therapie. Aktuell erfolgt die Probenvorbereitung für viele diagnostische Assays manuell (d.h. durch eine Abfolge aus manuell ausgeführten verschiedenen Pipettierschritten) oder kombiniert mit dem auszuführenden Assay in sogenannten Testkartuschen oder mittels Pipettierroboter. Die manuelle Durchführung der Probenvorbereitung ist zeitaufwendig und fehleranfällig und kann meist nur durch speziell geschultes Personal durchgeführt werden. Testkartuschen und Pipettierroboter ermöglichen eine automatisierte Durchführung der Prozessschritte, sind jedoch nur bedingt hinsichtlich des Durchsatzes skalierbar und bedürfen einer aufwendigen Steuerung durch externe Geräte.

Die Lab-on-a-Chip (LOC) Technologie bietet die Möglichkeit, die Probenvorbereitung durch Integration der verschiedenen Prozessschritte auf einer mikrofluidischen Plattform drastisch zu vereinfachen und zu automatisieren. Sie stellen damit eine Alternative zu Pipettierrobotern dar, die im Gegensatz zur Mikrofluidik größere Probenmengen verarbeiten. Die Mikrofluidik bietet den Vorteil, auch kleinste Probenmengen prozessieren und analysieren zu können. Dadurch, dass Assays in einem kompakten, miniaturisierten Format durchgeführt und die verschiedenen Prozessschritte auf einer Plattform integriert und parallelisiert werden, können Prozesszeiten und Kosten (weniger Prozessmedien, kein speziell geschultes Personal, kein teures Equipment) reduziert und die Prozesssicherheit erhöht werden. Die Technologie unterstützt die Portabilität und Point-of-Care Anwendung. Da in einem abgeschlossenen System gearbeitet wird, sind die Risiken für Kreuzkontaminationen und Gefahren für den Nutzer reduziert.

Da jedoch eine Abfolge von verschiedenen Prozessschritten für die Probenvorbereitung realisiert werden muss, ist die Komplexität aktueller Systeme vergleichsweise hoch. So werden im einfachsten Fall die Prozessmedien sequentiell appliziert oder es werden unter anderem pneumatische Elemente für den sequentiellen Fluidtransport genutzt (z. B. FilmArray der Firma BioFire). Dies bedarf zusätzlicher Geräte für die Ansteuerung dieser Elemente.

Im Stand der Technik sind mikrofluidische Systeme zur Probenvorbereitung bekannt. Kommerzielle Produkte bestehen in der Regel aus einem Einmalartikel/Einwegartikel (Artikel, die ein einziges Mal verwendet werden), welcher mit den notwendigen Reagenzien vorbeladen ist, und einem transportierbaren Gerät, dass die verschiedenen Prozessschritte automatisiert durchführt.

Der FilmArray der Firma BioFire besteht aus einem spritzgegossenen Polypropylen-Reservoir, welches die Biochemikalien enthält und einem aus Polyester/Polypropylene bestehenden Bereich, in welchen die mikrofluidischen Strukturen, wie Kanäle und "Blister" strukturiert werden. Dabei enthalten die "Blister" zusätzliche Reagenzien. Keramische Partikel sind zur Zelllyse und magnetische Silica-Partikel zur Nukleinsäureextraktion integriert. Für eine Auswertung mittels Polymerase-Kettenreaktion (PCR) sind lyophilisierte Oligonukleotide integriert. Das dazugehörige Gerät enthält zwei Peltier-Elemente, um das Temperaturprogramm der PCR zu realisieren und drei pneumatische Elemente, um den Transport der Fluide zu steuern. Kolben drücken einen Stempel in die Polypropylen-Reservoire und injizieren damit die Reagenzien in die mikrofluidischen Strukturen. Siliconblasen, die mittels der "Blister" bewegt werden, transportieren die Flüssigkeiten. Kolben, die über den Kanälen zwischen den "Blistern" angeordnet sind, kontrollieren den Flüssigkeitstransport zwischen den "Blistern". Dabei werden die Kolben, Siliconblasen und Dichtungen durch elektronisch gesteuerte Ventile aktiviert, um den Flüssigkeitsstrom entsprechend des Prozessprotokolls zu regulieren.

Die ID NOW Testplattform der Firma Abbott besteht aus einem Probenempfänger, einer Testbasis, einer Transferkassette und dem ID NOW-Gerät. Der Probenempfänger enthält bereits Elution/Lysepuffer. Die Testbasis besitzt zwei verschlossene Reaktionszylinder mit den entsprechenden lyophilisierten Pellets für die Amplifikation (für Probe und interne Kontrolle) und die Transferkassette ist für den Transport der eluierten Probe zur Testbasis verantwortlich. Das Gerät übernimmt Heiz- und Mischschritte sowie die Detektion. Die Durchführung des Assays erfolgt durch drei aufeinanderfolgende Schritte.

US10814321 offenbart eine Methode und eine molekular-diagnostische Vorrichtung zur Detektion, Analyse und Identifikation von genomischer DNA. Die Kartusche, die dem Transport von Proben mit genomischen Material dient, besteht aus einem Separations- und Direktionssystem. Der Transport der verschiedenen Reagenzien und der Probe wird u. a. durch Kapillarkräfte, durch Drücken sogenannter "Drückventile/Schubventile", durch Anlegen eines Vakuums oder durch elektrokinetische Kräfte realisiert. Die Isolation des genomischen Materials erfolgt in der genannten Erfindung mit Hilfe eines Substrates, welches das genomische Material binden und freigeben kann ("charge switch technology").

US20200215544 offenbart eine eigenständige Apparatur zur Isolierung von Nukleinsäuren, Zelllysaten und Zellsuspensionen, die mit einem Instrument verwendet wird. Die Apparatur besteht aus einer makroskopischen Komponente, die eine Kammer für die unbehandelte Probe enthält und Vorratsreservoire für die Reagenzien. Die mikrofluidische Komponente, die in Verbindung mit der makroskopischen Komponente steht, enthält die Matrix zur Nukleinsäurereinigung. Zudem verfügt die Apparatur über mindestens eine Schnittstelle zum Antriebsmechanismus am Instrument, um die Reagenzien durch das mikrofluidische Element und die Matrix zur Nukleinsäurereinigung zu treiben. Dabei erfolgt der Antrieb pneumatisch. Zur Fluidsteuerung können aktive oder passive Ventile verwendet werden, dabei ist die pneumatische Steuerung bevorzugt.

US10947528 offenbart eine mikrofluidische Vorrichtung zur Extraktion, Isolation und Analyse von DNA aus Zellen. Diese umfasst eine Einlassöffnung, um die Probe aufzunehmen, eine Auslassöffnung, um die isolierte DNA zu erhalten und einen mikrofluidischen Kanal, der ein Mikropfostenarray zum Einfangen von Zellen sowie zum Binden von DNA umfasst. Die Zellen und Reagenzien werden durch einen druckgesteuerten Fluss in das mikrofluidische System eingebracht, wobei die Spritze entsprechend mit den Reagenzien nachgefüllt wird.

US10843188 offenbart ein integriertes System zur Prozessierung von mikrofluidischen Proben und eine Methode zur Nutzung des Systems. Es umfasst einen Apparat und eine mikrofluidische Kartusche, die Reagenzien und ein mikrofluidisches Netzwerk zur Prozessierung der Probe enthält, wobei das mikrofluidische Netzwerk auch eine Anordnung zur Rückhaltung der Polynukleotide (Nukleinsäuren) enthält. Diese Rückhalteanordnung umfasst ein Polyalkylenimin oder ein polykationisches Polyamid in Form eines oder mehrerer Partikel, die der mikrofluidischen Kartusche auch entnommen werden können. Die Fluidsteuerung erfolgt durch integrierte Phasenwechselmaterialien, wie z. B. eutektische Legierungen, Wachs, Polymere, Kunststoffe und deren Kombinationen. Zum Transport von Flüssigkeiten werden Aktuatoren verwendet, die einen Gasdruck erzeugen.

US11142757 offenbart eine mikrofluidische Kartusche zur Prozessierung und Detektion von Nukleinsäuren. Dabei erfolgt die Extraktion der Nukleinsäuren mittels magnetischer Partikel. Durch eine Reihe von externen Stiften oder anderen Vertiefungen, die durch Ventilführungen geleitet werden, wird der Fluss durch einen mikrofluidischen Kanal beeinflusst und die Fluidsteuerung realisiert.

US10940473 offenbart eine mikrofluidische Vorrichtung zur Nukleinsäureanalyse. Diese umfasst zumindest eine erste mikrofluidische Kammer und zumindest einen ersten mikrofluidischen Kanal, um die Probe zu transportieren. Ein zweiter mikrofluidischer Kanal transportiert die Lysechemikalien zur ersten mikrofluidischen Kammer, wobei die Isolation und Reinigung der Nukleinsäuren mittels Affinitätsbeads erfolgt. Die Fluidsteuerung erfolgt über elastische Ventile, wobei das Anlegen eines Druckes (Gas oder Flüssigkeit) an einen fluidischen Kanal zu einer Auslenkung einer elastischen Membran führt, welche den Fluidstrom in einem darunterliegenden Kanal blockiert bzw. behindert.

Die EP2836302 offenbart ein Verfahren und eine Vorrichtung zur gezielten Prozessführung in einem mikrofluidischen System mit integrierten aktiven Elementen, um volumetrisch definierte Mischungsreaktionen in definierten Zeitabläufen durchzuführen. Dabei wird eine Kammer, die durch eine lösliche Membran getrennt ist, parallel von zwei oder mehreren Flüssigkeiten befüllt. Durch die integrierten aktiven Elemente erfolgen die Volumendefinition und zeitliche Definition der Mischungsreaktion. Das offenbarte Verfahren und die Vorrichtung erlauben damit eine gezielte Prozessführung durch integrierte aktive Elemente, die durch Interaktion mit den Prozessmedien einen Fluidstrom erlauben oder unterbinden. Dabei wird jedoch lediglich das Verfahren zum Vermischen einer ersten und einer zweiten Flüssigkeit offenbart. Die Transportkanäle der Vorrichtung sind hierfür parallel angeordnet und münden in einer Mischkammer. Die Vorrichtung ermöglicht jedoch nicht die sequentielle Bereitstellung von Fluiden in einer Mikrofluidischen Vorrichtung. Ebenso wenig wird ein Verfahren zur sequentiellen Abfolge einer Prozessführung zur Probenvorbereitung bereitgestellt. Zudem sind in diesem Patent keine Komponenten beschrieben, die das Anbinden zumindest eines Bestandteils der Probe erlauben (Immobilisierung von Bestandteilen einer Probe).

Die DE112007003160 offenbart einen automatischen Mikrofluidik-Prozessor mit integrierten aktiven Elementen, der durch logische Zusammenschaltung der aktiven Einzelelemente Teilaufgaben einer definierten Prozedur zu einer Aufgabensequenz verknüpft und die Aktivierungszeitpunkte sowie weitere Parameter der Einzelelemente durch das Prozessor-Design festlegt. Dadurch sollen mehr oder minder aufwändige biologische, biochemische oder chemische Prozesse auf einem automatischen Mikrofluidik-Prozessor realisiert werden. Die offenbarte Vorrichtung erlaubt durch logische Zusammenschaltung der aktiven Bauelemente zwar die Abarbeitung von Teilprozeduren, die Bestandteil von z. B. chemischen, biochemischen oder biologischen Prozessen sind. Jedoch werden dabei lediglich Prozesse zum Nachweis von z. B. biologischen Komponenten ermöglicht, aber kein Verfahren zur Probenvorbereitung. Zudem ist die Integration eines Fangbereiches zur z. B. Anbindung von Nukleinsäuren nicht beschrieben.

Die Aufgabe der vorliegenden Erfindung besteht daher darin, eine Vorrichtung und ein Verfahren bereitzustellen, die dazu dienen, ein komplexes, mehrstufiges Protokoll zur Probenvorbereitung automatisiert ohne externe Steuerung durchzuführen.

Hierfür stellt die Erfindung eine Vorrichtung gemäß Anspruch 1 und ein Verfahren gemäß Anspruch 12 bereit.

**Beschreibung**

Die erfindungsgemäße fluidische Vorrichtung weist einen ersten Kanalabschnitt und *n* zweite Kanalabschnitte, wobei $n \in \mathbb{N}$ und *n* ≥ 1, mindestens ein Öffnerelement und mindestens ein Schließerelement auf.

Erfindungsgemäß ist jeder der n zweiten Kanalabschnitte durch ein Öffnerelement angrenzend an den ersten Kanalabschnitt angeordnet. In einer Ausführungsform ist jeder der n zweiten Kanalabschnitte peripher zum ersten Kanalabschnitt angeordnet. Insbesondere ist in einer Ausführungsform jeder der zweiten Kanalabschnitte im Bereich des Öffnerelementes annähernd parallel zum ersten Kanalabschnitt angeordnet. Jedes der Öffnerelemente ist erfindungsgemäß dazu eingerichtet, eine fluidische Verbindung von dem jeweiligen zweiten Kanalabschnitt zum ersten Kanalabschnitt herzustellen. Weiterhin weisen der erste und die *n* zweiten Kanalabschnitte jeweils einen Aufnahmebereich auf. Das heißt der erste Kanalabschnitt weist einen Aufnahmebereich auf und jeder der *n* zweiten Kanalabschnitt weist ebenfalls je einen Aufnahmebereich auf.

Erfindungsgemäß ist im ersten Kanalabschnitt in Strömungsrichtung eines Fluids mindestens einem der Öffnerelemente ein Schließerelement vorgelagert angeordnet. In einer Ausführungsform der Erfindung ist in Strömungsrichtung eines Fluids jeweils ein Schließerelement vor jedem Öffnerelement im ersten Kanalabschnitt angeordnet.

Die Strömung im ersten Kanalabschnitt bildet sich vom Aufnahmebereich in Richtung des Schließerelementes im ersten Kanalabschnitt aus und setzt sich entlang des ersten Kanalabschnittes fort. Unter Strömungsrichtung eines Fluids im ersten Kanalabschnitt wird erfindungsgemäß daher die Strömung des Fluids ausgehend vom Aufnahmebereich hin zum Schließerelement und darüber hinaus entlang des ersten Kanalabschnittes verstanden. In jedem zweiten und jedem weiteren Kanalabschnitt bildet sich die Strömung eines Fluids vom Aufnahmebereich des zweiten oder des weiteren Kanalabschnittes in Richtung des Öffnerelementes in dem zweiten oder weiteren Kanalabschnitt aus und setzt sich entlang des zweiten oder weiteren Kanalabschnittes fort. Unter Strömungsrichtung eines Fluids in jedem zweiten und jedem weiteren Kanalabschnitt wird erfindungsgemäß daher die Strömung des Fluids ausgehend vom Aufnahmebereich des zweiten oder des weiteren Kanalabschnittes hin zum Öffnerelement und darüber hinaus entlang des zweiten oder des weiteren Kanalabschnittes verstanden.

In einer Ausführungsform folgt auf den Aufnahmebereich des ersten Kanalabschnittes im ersten Kanalabschnitt ein Schließerelement und anschließend ein Öffnerelement, durch welches ein zweiter Kanalabschnitt an den ersten Kanalabschnitt angrenzt. Weitere zweite Kanalabschnitte können nachfolgend über weitere Öffnerelemente am ersten Kanalabschnitt angrenzend angeordnet sein. In einer Ausführungsform ist dabei zwischen jedem der weiteren Öffnerelemente im ersten Kanalabschnitt jeweils ein weiteres Schließerelement angeordnet.

Der Begriff Kanalabschnitt umfasst sowohl rechteckige Kanäle als auch Kanäle mit einem kreisförmigen Querschnitt (Schläuche). Die Kanaldurchmesser liegen üblicherweise im Bereich von 50 μm bis 1 mm, bevorzugt im Bereich von 100 μm bis 500 μm, besonders bevorzugt im Bereich von 150 μm bis 300 μm. Kanäle mit eckigen Querschnitten weisen entsprechend Dimensionen auf, die mit den beschriebenen Kanaldurchmessern vergleichbar sind. Kanalabschnitte im Sinne der Erfindung sind keine Misch- oder Reaktionskammern, in denen eine Mischreaktion zwischen verschiedenen Fluiden stattfindet. Insbesondere findet keine signifikante und damit funktionsrelevante Vermischung von Fluiden aus dem ersten und/oder aus zweiten Kanalabschnitten im ersten Kanalabschnitt statt. Die Kanalabschnitte dienen dem sequentiellen Transport von Fluiden in der erfindungsgemäßen Vorrichtung.

In einer Ausführungsform weist die fluidische Vorrichtung n ≥ 2 zweite Kanalabschnitte auf. Jeder der n zweiten Kanalabschnitte ist erfindungsgemäß beabstandet voneinander in Reihe am ersten Kanalabschnitt angeordnet. Darunter ist zu verstehen, dass die *n* zweiten Kanalabschnitt in Form einer Reihenschaltung am ersten Kanalabschnitt angeordnet sind. Beabstandet oder auch räumlich beabstandet beschreibt erfindungsgemäß, dass die zweiten Kanalabschnitte hintereinander in Strömungsrichtung eines Fluids im ersten Kanalabschnitt am ersten Kanalabschnitt angeordnet sind. Grenzt beispielsweise ein erster zweiter Kanalabschnitt an den ersten Kanalabschnitt an, so ist der nächste angrenzende zweite Kanalabschnitt in Strömungsrichtung eines Fluids im ersten Kanalabschnitt räumlich beabstandet vom ersten zweiten Kanalabschnitt am ersten Kanalabschnitt angeordnet. Dies gilt analog für jeden weiteren zweiten Kanalabschnitt. Besonders bevorzugt entstehen keine Überlagerungsbereiche zwischen verschiedenen zweiten Kanalabschnitten. Das heißt, ein Fluid aus einem ersten zweiten Kanal und ein Fluid aus einem weiteren zweiten Kanalabschnitt werden zeitlich getrennt, also sequentiell durch den ersten Kanalabschnitt geleitet. An den Grenzflächen aufeinanderfolgender Fluide kann es dabei zwar zu einer leichten Vermischung durch Diffusion oder Turbulenzen kommen, die jedoch so gering wie möglich sein sollte und keinesfalls einen vollständigen Mischvorgang zweier Fluide darstellt, wie er in einer Mischkammer abläuft. Insbesondere findet erfindungsgemäß damit keine signifikante und damit funktionsrelevante Vermischung von Fluiden aus dem ersten und/oder aus dem zweiten Kanalabschnitten im ersten Kanalabschnitt statt.

Die erfindungsgemäße fluidische Vorrichtung ist bevorzugt dazu eingerichtet, Fluide sequentiell durch den ersten Kanalabschnitt zu transportieren.

In einer Ausführungsform weist die fluidische Vorrichtung weiterhin *m* dritte Kanalabschnitte auf weist, wobei $m \in \mathbb{N}$ und m ≥ 1, wobei jeder der *m* dritten Kanalabschnitte jeweils durch ein Öffnerelement angrenzend an zumindest einen der *n* zweiten Kanalabschnitte angeordnet ist. Das jeweilige Öffnerelement ist erfindungsgemäß dazu eingerichtet, eine fluidische Verbindung von einem dritten Kanalabschnitt zu einem zweiten Kanalabschnitt herzustellen. Besonders bevorzugt weist jeder der *m* dritten Kanalabschnitte einen eigenen Aufnahmebereich auf.

In einer weiteren Ausführungsform weist die fluidische Vorrichtung weiterhin *f k*-te Kanalabschnitte auf, wobei $f \in \mathbb{N}$ und *f* ≥ 1 sowie $k \in \mathbb{N}$ und *k* ≥ 4. Jeder der *f k*-ten Kanalabschnitte ist jeweils durch ein Öffnerelement angrenzend an zumindest einen der *(k-1)*-ten Kanalabschnitte angeordnet, wobei das jeweilige Öffnerelement dazu eingerichtet ist, eine fluidische Verbindung von einem *k*-ten Kanalabschnitt zu einem *(k-1)*-ten Kanalabschnitt herzustellen. In dieser Ausführungsform kann die fluidische Vorrichtung damit beispielsweise *f* vierte Kanalabschnitte aufweisen, wobei jeder der *f* vierten Kanalabschnitte jeweils durch ein Öffnerelement an einen dritten Kanalabschnitt angrenzt. Im Sinne der Erfindung können darüber hinaus weiterhin *f* fünfte Kanalabschnitte vorhanden sein, wobei jeder der *f* fünften Kanalabschnitte jeweils durch ein Öffnerelement an einen vierten Kanalabschnitt angrenzt usw. Besonders bevorzugt weist jeder der *f k*-ten Kanalabschnitte einen eigenen Aufnahmebereich auf.

In einer Ausführungsform ist *k* bevorzugt 4, 5, 6, 7, 8, 9 oder 10.

Alle Eigenschaften, die für die *n* zweiten Kanalabschnitte beschrieben sind, treffen genauso auf die *m* dritten und *f k*-ten Kanalabschnitte zu.

Die Schließerelemente und Öffnerelemente sind derart ausgebildet, dass sie jeweils einen geöffneten Zustand und einen geschlossenen Zustand einnehmen können. Öffner- und Schließerelemente übernehmen in der vorliegenden Erfindung die Funktion von Ventilen. Im geöffneten Zustand ist das Durchströmen des geöffneten Öffnerelementes oder Schließerelementes durch ein Fluid möglich. Das geöffnete Öffnerelement oder Schließerelement wirkt wie ein geöffnetes Ventil. Im geschlossenen Zustand des Öffnerelementes oder Schließerelementes ist ein Durchströmen des Öffnerelementes oder Schließerelementes durch ein Fluid nicht möglich. Das geschlossene Öffnerelement oder Schließerelement stellt damit eine Barriere dar und wirkt wie ein geschlossenes Ventil.

<u>Schließerelement</u>

In einer bevorzugten Ausführungsform der vorliegenden Erfindung sind die Schließerelemente als Quellmittelbarrieren ausgeführt, die durch Fluidkontakt aktivierbar sind. Bei Benetzung der Schließerelemente mit einem Fluid im Strömungsweg erfolgt durch eine Fluidaufnahme eine Volumenzunahme des Schließerelementes, wodurch der Strömungsweg in dem Kanalabschnitt, in dem das Schließerelement angeordnet ist, immer weiter verengt wird, bis es infolge der vollständigen Ausfüllung des Querschnitts des Kanalabschnittes zu einem Strömungsabriss im Strömungsweg und mithin zu einer Unterbrechung der Strömung kommt.

Das als Quellmittelbarriere ausgeführte Schließerelement wird in trockenem Zustand in einen Kanalabschnitt der fluidischen Vorrichtung eingebracht. Nach erfolgter Volumenzunahme des Schließerelementes durch Fluidaufnahme verbleibt das Schließerelement und damit die Quellmittelbarriere im gequollenen Zustand. Das bedeutet, dass nach Volumenzunahme keine Entquellung stattfindet, wodurch die Quellmittelbarriere nur eine einmalige Aktivierung durch die Fluidaufnahme erfährt. Dies ist insbesondere vorteilhaft, da durch das Schließerelement beispielsweise das Volumen eines Fluids in der mikrofluidischen Vorrichtung definiert werden kann.

In einer Ausführungsform der Erfindung weisen die Schließerelemente Hydrogele auf oder bestehen aus diesen. Bevorzugt sind die Hydrogele chemisch und/oder physikalisch vernetzbar. Unter Hydrogelen wird im Sinne der Erfindung ein Wasser enthaltendes, aber wasserunlösliches Polymer verstanden, dessen Moleküle chemisch, z. B. durch kovalente Bindungen, oder physikalisch, z. B. durch Verschlaufen der Polymerketten, zu einem dreidimensionalen Netzwerk verknüpft sind. Durch eingebaute hydrophile Polymerkomponenten quellen sie in Flüssigkeiten unter beträchtlicher Volumenzunahme, ohne aber ihren stofflichen Zusammenhalt zu verlieren. Erfindungsgemäß sind die Hydrogele derart ausgebildet, dass diese nach Kontakt mit Fluiden im gequollenen Zustand verbleiben.

In einer weiteren Ausführungsform der Erfindung weisen die Schließerelemente Hydrogele auf, welche ausgewählt sind aus einer Gruppe, aufweisend Polyacrylamide, Polyvinylalkohole, Polyacrylate, Hydroxycellulose, Polyvinylpyridine oder Polyglykole (z. B. Polyethylenglykol, Polypropylenglykol) und deren Derivate oder bestehen aus diesen.

Unter dem Aktivieren des Schließerelementes wird im Sinne der Erfindung verstanden, dass das Schließerelement vom geöffneten in den geschlossenen Zustand übergeht. Die Aktivierung beginnt mit dem Kontakt des Schließerelementes mit einem Fluid, welches das Quellen des Schließerelementes initiiert. Die Aktivierungszeit beschreibt den Zeitraum ab der Aktivierung bis das Schließerelement so weit gequollen ist, dass der Strömungsweg durch das Schließerelement vollständig verschlossen ist. Ist das Schließerelement als Quellmittelbarriere ausgeführt, kann dieses durch den Kontakt mit einem Fluid aktiviert werden, welches zum Aufquellen des Schließerelementes führt. Vorteilhafterweise kann die Zeit, die das Schließerelement benötigt, um vom geöffneten in den geschlossenen Zustand überzugehen, durch

- die verwendeten Materialien und deren Zusammensetzung; und/oder
- die Geometrie des Schließerelementes; und/oder
- die Art des Fluids welches das Schließerelement aktiviert; und/oder
- den Druck bzw. den Volumenstrom im Kanalabschnitt; und/oder
- die Temperatur; und/oder
- die Geometrie des umgebenden Kanalabschnittes bzw. des umgebenden Strukturträgers, der den Kanalabschnitt bildet,

exakt definiert werden.

Der Einfluss der Geometrie des umgebenden Kanalabschnittes bzw. des umgebenden Strukturträgers, der den Kanalabschnitt bildet, wird durch die Sitzgröße beschrieben. Das Schließerelement ist umgeben von Wänden eines Kanals und/oder Strukturträgers, die einen Freiraum definierter Größe zur Verfügung stellen, in den das Schließerelement eingebracht wird. Der Freiraum, in den das Schließerelement eingebracht wird, wird als Sitzgröße bezeichnet. Bevorzugt sind die Maße des Schließerelementes im gequollenen Zustand größer als die Maße der Sitzgröße, so dass das Schließerelement an die Kanalwände bzw. Wände des Strukturträger im gequollenen Zustand angepresst wird und damit den Kanalabschnitt verschließt.

Weist das Schließerelement Hydrogele auf, so können in einer bevorzugten Ausführungsform die Schließerelemente aus den Hydrogelen in gequollener Form hergestellt werden. Hierfür werden Formen verwendet, deren Durchmesser und Höhe größer als die später verwendete Sitzgröße (Ventilsitzdurchmesser und Kanalhöhe) ist. Nach dem Trocknen dieser Schließerelemente ist deren Durchmesser und Höhe geringer als die Sitzgröße und die Schließerelemente können unproblematisch in einen Kanalabschnitt eingebracht werden. Entsprechend kann die Geometrie des Schließerelementes bei der Herstellung verändert werden.

In einer Ausführungsform wird insbesondere durch die Variation der Sitzgröße die Zeit, die ein Schließerelement benötigt, um vom geöffneten in den geschlossenen Zustand überzugehen, verändert.

Öffnerelemente

In einer bevorzugten Ausführungsform sind Öffnerelemente als lösliche Barrieren ausgebildet, die als fluidlösliche Ventile dienen. Durch die Benetzung des Öffnerelementes mit einem Fluid im Strömungsweg wird eine Auflösung der Barriere erzielt. Durch die Auflösung der Barriere kommt es zu einem Ansteigen der Durchströmung und ein erster Strömungsweg in einem Kanalabschnitt und ein zweiter Strömungsweg in einem weiteren Kanalabschnitt werden fluidisch miteinander verbunden.

In einer weiteren Ausführungsform der Erfindung sind die Öffnerelemente aus unvernetzten Polymeren, Salzen oder organischen Naturstoffen wie Sacchariden ausgeführt. Dies ist der Fall, wenn die aktiven Elemente als flüssigkeitslösliche Barrieren ausgeführt sind. Dabei können sämtliche Materialien eingesetzt werden, die im getrockneten Zustand einen Feststoff, Sol-Gel oder dergleichen bilden und bei Kontakt mit einer Flüssigkeit in Lösung gehen. Die Materialbasis der unvernetzten Polymere kann prinzipiell die gleiche wie bei den vernetzten Polymeren der Schließerelemente sein. Während die zu einem dreidimensionalen Netzwerk vernetzten Polymere als quellbare Quellmittelbarrieren dienen, lösen sich die gleichen Polymere in der Flüssigkeit auf, wenn sie unvernetzt sind, da die nicht miteinander verbundenen Polymerketten in Lösung gehen können.

In einer Ausführungsform der Erfindung weisen die Öffnerelemente lösliche Polymere auf, welche ausgewählt sind aus einer Gruppe, aufweisend Polyvinylalkohole, Hydroxycellulose, oder Polyglykole (z. B. Polyethylenglykol, Polypropylenglykol) und deren Derivate oder bestehend aus diesen.

In einer weiteren Ausführungsform der Erfindung weisen die Öffnerelemente Polyglykole auf, Mischungen von Polyglykolen verschiedener Kettenlängen oder Mischungen von Polyglykolen verschiedener Kettenlänge und Zusätzen, wie z. B. Mannose oder Zellulose-Fasern.

In einer Ausführungsform weist das Öffnerelement mindestens ein Paar von Verbindungselementen und einen kanalförmigen Teil auf. Das Paar von Verbindungselementen besteht aus einem ersten und einem zweiten Verbindungselement, die eine Fluidverbindung zwischen einem ersten Kanalabschnitt und einem zweiten Kanalabschnitt erzeugen, wenn sich das Öffnerelement im geöffneten Zustand befindet. Dabei wird durch das erste Verbindungselement eine fluidische Verbindung zwischen einem ersten Kanalabschnitt und dem Öffnerelement erzeugt und durch das zweite Verbindungselement eine fluidische Verbindung zwischen einem zweiten Kanalabschnitt und dem Öffnerelement. Die Verbindungselemente dieser Ausführungsform der Öffnerelemente treten daher immer als Paar von Verbindungelementen auf.

Jedes Verbindungselement des Paars von Verbindungselementen stellt dabei mindestens eine plötzliche Ausdehnung des Öffnerelementes in Richtung eines Kanalabschnittes dar, wobei eine plötzliche Ausdehnung erfindungsgemäß eine kanalförmige Verbindung zu einem Kanalabschnitt sein kann.

Jede plötzliche Ausdehnung eines Verbindungselementes grenzt erfindungsgemäß an einen Kanalabschnitt an. Die Außenwand der plötzlichen Ausdehnung bildet dabei erfindungsgemäß zur Außenwand des angrenzenden Kanalabschnittes einen sogenannten Öffnungswinkel $\alpha$. Erfindungsgemäß ist der Öffnungswinkel $\alpha \leq 90°$.

In einer Ausführungsform mit Verbindungselementen ist weiterhin mindestens eine plötzliche Ausdehnung eines Verbindungselementes orthogonal zum kanalförmigen Abschnitt des Öffnerelementes ausgerichtet.

In einer Ausführungsform der vorliegenden Erfindung weist der kanalförmige Teil des Öffnerelementes einen kammerförmigen Teil auf. Der kammerförmige Teil kann beispielsweise die Form eines Quaders, Zylinders oder eines elliptischen Zylinders aufweisen. Die Höhe des kammerförmigen Teils entspricht dabei der Höhe des kanalförmigen Teils des Öffnerelementes. In dieser Ausführungsform grenzt die mindesten eine plötzliche Ausdehnung und jede weitere plötzliche Ausdehnung eines Verbindungselementes an die kammerförmige Ausdehnung des kanalförmigen Teils des Öffnerelementes an.

In einer Ausführungsform der vorliegenden Erfindung weist ein Verbindungselement mehrere plötzliche Ausdehnungen auf. Ein Verbindungselement weist beispielsweise 1 bis 30, bevorzugt 1 bis 20, besonders bevorzugt 1 bis 10 plötzliche Ausdehnungen auf. Bevorzugt weist jede plötzliche Ausdehnung die gleiche Geometrie auf.

Besonders bevorzugt ist, dass die mindestens zwei Verbindungselemente, die ein Paar von Verbindungselementen bilden und damit eine fluidische Verbindung zwischen einem ersten und einem zweiten Kanalabschnitt herstellen, die gleiche Anzahl plötzlicher Ausdehnungen aufweisen.

In einer Ausführungsform der vorliegenden Erfindung sind das erste und zweite Verbindungselement eines Paares von Verbindungelementen, gegenüberliegend zueinander angeordnet. Besonders bevorzugt sind darüber hinaus sämtliche plötzliche Ausdehnungen des ersten Verbindungselementes gegenüberliegend zu jeweils einer plötzlichen Ausdehnung des zweiten Verbindungselementes, also paarweise, angeordnet. In einer weiteren Ausführungsform sind das erste und zweite Verbindungselement eines Paares von Verbindungelementen parallel zueinander angeordnet.

Unter dem Aktivieren des Öffnerelementes wird im Sinne der Erfindung verstanden, dass das Öffnerelement vom geschlossenen in den geöffneten Zustand übergeht. Ist das Öffnerelement als lösliche Barriere ausgeführt, kann dieses durch den Kontakt mit einem Fluid aktiviert werden, welches zum Auflösen des Öffnerelementes führt. Die Aktivierung beginnt mit dem Kontakt des Öffnerelementes mit einem Fluid, welches das Öffnen, beispielsweise durch Auflösen einer löslichen Barriere, des Öffnerelementes initiiert. Die Aktivierungszeit beschreibt den Zeitraum ab der Aktivierung bis das Öffnerelement geöffnet ist, so dass der Strömungsweg durch das Öffnerelement geöffnet ist. Vorteilhafterweise kann die Zeit, die das Öffnerelement benötigt, um vom geschlossenen in den geöffneten Zustand überzugehen, durch

- die verwendeten Materialien und deren Zusammensetzung und/oder
- der Geometrie des Öffnerelementes; und/oder
- die Art des Fluids, welches das Öffnerelement aktiviert; und/oder
- den Druck bzw. den Volumenstrom im Kanalabschnitt; und/oder
- die Temperatur; und/oder
- die Geometrie des umgebenden Kanalabschnittes bzw. des Strukturträgers, der einen Kanalabschnitt bildet, zum Beispiel in Form der Stufenbreite

exakt definiert werden.

Das Öffnerelement ist umgeben von Wänden eines Kanals und/oder Strukturträgers, die einen Freiraum definierter Größe (z. B. Loch, Kavität) zur Verfügung stellen, in die das Öffnerelement eingebracht ist. In einer Ausführungsform kann die Zeit, die das Öffnerelement benötigt, um vom geschlossenen in den geöffneten Zustand überzugehen durch den Lochdurchmesser und die Höhe des Öffnerelement (z. B. vorgegeben durch die Höhe des Strukturträgers oder die Dicke der Membran, wenn das Öffnerelement als Membran ausgestaltet ist) bzw. die Stufenbreite beeinflusst werden. Je dicker das Öffnerelement bzw. je breiter die Stufe, desto mehr Material weist das Öffnerelement auf und desto mehr Zeit wird für das Auflösen der Barriere benötigt.

Sowohl die Öffnerelemente als auch die Schließerelemente gemäß der vorliegenden Erfindung werden damit hilfsenergiefrei aktiviert. Unter hilfsenergiefrei wird im Sinne der vorliegenden Erfindung der Verzicht auf die Zuführung von Energie aus einer extern gesteuerten elektrischen oder thermischen Energiequelle zu den erfindungsgemäßen Öffner- und Schließerelementen verstanden. Aufgrund der zeitlich definierbaren und hilfsenergiefreien Funktionsausübung der erfindungsgemäßen Öffner- und Schließer-elemente eignen sich diese vorteilhafterweise für den Einsatz als Ventile in autarken mikrofluidischen Vorrichtungen.

Durch die Variation des Aufbaus von Öffner- und Schließerelementen kann wie bereits beschrieben direkt Einfluss auf die zeitliche Abfolge der fluidischen Kontaktierung sowie die Dosierung der Fluide in einer mikrofluidischen Vorrichtung genommen werden. Dabei können beispielsweise durch geeignete Auswahl an Materialien und der Dimensionierung der Öffner- und Schließerelemente das Zeitverhalten in der fluidischen Vorrichtung beeinflusst werden.

Die Zahl der Kanalabschnitt und damit der Öffner- und Schließerelemente in der fluidischen Vorrichtung hängt von der Komplexität der Anwendung, beispielsweise der verwendeten Assays ab. Dabei spielt vor allem die Anzahl der notwendigen fluidischen Verfahrensschritte eine entscheidende Rolle.

Fangelement

In einer Ausführungsform der vorliegenden Erfindung weist der erste Kanalabschnitt weiterhin ein Fangelement auf, welches in Strömungsrichtung eines Fluids nach dem letzten Öffnerelement, welches einen der n zweiten Kanalabschnitte mit dem ersten Kanalabschnitt verbindet, im ersten Kanalabschnitt angeordnet ist.

Im Sinne der Erfindung wird unter Fangelement ein Bereich verstanden, der den Strömungsweg im ersten Kanalabschnitt vor dem Fangelement über ein permeables Element mit dem Strömungsweg im ersten Kanalabschnitt nach dem Fangelement verbindet, wobei die beiden Strömungswege im Fangelement über eine gemeinsame Wandung verfügen.

In einer bevorzugten Ausführungsform ist das Fangelement ein permeables Element. Diese kann in einer Ausführungsform als poröses Element mit mehreren Öffnungen ausgebildet sein. Geeignete poröse Elemente sind ausgewählt aus der Gruppe aufweisend Filter, poröse Membranen oder eine Gelmatrix. Diese bieten zahlreiche Öffnungen in Form von Poren und/oder Kanälen. In einer bevorzugten Ausführungsform werden Zellulosemembranen, Silikamembranen oder integrierte Hydrogelpartikel verwendet.

Als integrierte Hydrogelpartikel können die Hydrogelpartikel in einer Ausführungsform als Schüttgut im ersten Kanalabschnitt platziert werden, wobei erste Kanalabschnitt unmittelbar vor und nach dem Schüttgut derart verengt wird, dass das Schüttgut an dem dafür vorgesehenen Platz bleibt und das Fangelement bildet. In einer weiteren Ausführungsform können die Hydrogelpartikel auch in einem mehrlagigen Aufbau zwischen zwei begrenzenden Netzen angeordnet werden und so das Fangelement bilden.

In einer Ausführungsform kann das Fangelement beispielsweise als Bulkmaterial ausgebildet sein oder auch als Pfosten. Die Pfosten können als Hydrogelstrukturen ausgebildet sein oder als Silikapfosten vorliegen oder aus anderen funktionalisierten Strukturen wie beispielsweise PMMA gebildet werden.

Durch das Fangelement können fluidische Proben hindurchtreten, die beispielsweise Polynukleotide, Proteine, Lipide oder Kohlenhydrate aufweisen. Dabei kann die Oberflächen des permeablen Elements erfindungsgemäß so ausgewählt bzw. modifiziert werden, dass Polynukleotide, Proteine, Lipide oder Kohlenhydrate bevorzugt zurückgehalten werden. Dem Fachmann vertraut sind beispielsweise Filtermembranen, die aus Cellulose bestehen und bevorzugt zum Zurückhalten von Nukleinsäuren verwendet werden können.

In einer Ausführungsform ist unmittelbar vor dem Fangelement ein Schließerelement angeordnet. Unmittelbar bedeutet, dass zwischen dem Fangelement und dem Schließerelement keine weiteren Komponenten, insbesondere kein Öffnerelement, angeordnet sind.

Aufnahmebereich

Jeder Kanalabschnitt verfügt über genau einen Aufnahmebereich, sowohl der Erste, als auch die n zweiten, m dritten Kanalabschnitte und f k-ten Kanalabschnitte. Über den jeweiligen Aufnahmebereich erfolgt die Bereitstellung der Prozessmedien.

In einer bevorzugten Ausführungsform ist jeder Aufnahmebereich so ausgestaltet, dass er zur Probenaufgabe dienen kann oder als Reservoir für Prozessmedien oder als Mischerstruktur.

Ein Reservoir für Prozessmedien kann beispielsweise durch die Bereitstellung eines Blisters mit dem jeweiligen Prozessmedium zur Verfügung gestellt werden. Der Blister wird mit dem Aufnahmebereich verbunden.

Weiterhin kann der Aufnahmebereich in Form einer Mischerstruktur ausgebildet sein. In dieser Mischerstruktur werden mindestens zwei Prozessmedien gemischt und gelangen anschließend in den zugehörigen Kanalabschnitt.

Abluftventil

In einer Ausführungsform weisen alle Kanalabschnitte außer dem ersten Kanalabschnitt ein Abluftventil auf.

Bekannte Abluftventile sind hydrophobe Membranen. In einer bevorzugten Ausführungsform weist das Abluftventil eine PTFE-Membran auf oder besteht aus dieser. Die Durchlässigkeit der PTFE-Membran (Polytetrafluorethylen-Membran) für wässrige Lösungen ist abhängig vom Druck im Kanalabschnitt und der Hydrophilie der Lösung. Mit steigendem Druck wird die PTFE-Membran dabei durchlässig für wässrige Lösungen.

In einer weiteren Ausführungsform der Erfindung ist das Abluftventil ein Schließerelement.

In einer weiteren Ausführungsform der Erfindung ist das Abluftventil eine Kombination aus einer hydrophoben Membran und einem Schließerelement.

In einer Ausführungsform der Erfindung ist jeder zweite, dritte und k-te Kanalabschnitt wie folgt aufgebaut. Der Kanalabschnitt weist an seinem einen Ende einen Aufnahmebereich auf, durch die ein Fluid in den Kanalabschnitt eingebracht werden kann. Das Fluid strömt durch den Kanalabschnitt, der an seinem anderen Ende ein Abluftventil aufweist, so dass die Luft aus dem Kanalabschnitt entweichen kann. Direkt vor dem Abluftventil ist ein Schließerelement angeordnet, welches durch den Fluidkontakt aktiviert wird und den Kanalabschnitt zum Abluftventil hin verschließt, so dass kein Fluid aus dem Abluftventil austreten kann. Diese Ausführungsform ist vorteilhaft, wenn ein Fluid genutzt wird, welches eine so geringe Hydrophilie aufweist, dass es durch eine hydrophobe Membran hindurchdringen kann oder wenn der Druck im Kanalabschnitt so hoch ist, dass das Fluid durch die hydrophobe Membran dringt.

In einer weiteren Ausführungsform der Erfindung ist jeder zweiter, dritte und k-te Kanalabschnitt wie folgt aufgebaut. Der Kanalabschnitt weist an seinem einen Ende einen Aufnahmebereich auf, durch die ein Fluid in den Kanalabschnitt eingebracht werden kann. Das Fluid strömt durch den Kanalabschnitt, der an seinem anderen Ende ein Abluftventil aufweist, so dass die Luft aus dem Kanalabschnitt entweichen kann. In dieser Ausführungsform ist das Abluftventil eine hydrophobe Membran, bevorzugt eine PTFE-Membran. Diese Ausführungsform ist besonders vorteilhaft, wenn ein Fluid genutzt wird dessen Hydrophilie ausreichend groß ist, dass es die hydrophobe Membran nicht durchdringen kann.

Abfallkammer

In einer weiteren Ausführungsform der vorliegenden Erfindung, weist die mikrofluidische Vorrichtung weiterhin mindestens eine Abfallkammer mit einem Abluftventil auf, wobei der erste Kanalabschnitt in die Abfallkammer mündet und wobei am Eingang der Abfallkammer im ersten Kanalabschnitt ein Schließerelement angeordnet ist. Das Abluftventil ist derart an der Abfallkammer angeordnet, dass durch das Abluftventil die Luft aus dem ersten Kanalabschnitt entweichen kann. Durch das Schließerelement am Eingang der Abfallkammer, kann diese zu einem definierten Zeitpunkt verschlossen werden, indem ein Fluid durch den ersten Kanalabschnitt strömt, welches geeignet ist das Schließerelement zu aktivieren.

In einer weiteren Ausführungsform weist die erfindungsgemäße Vorrichtung mehrere Abfallkammern auf, wobei jede weitere Abfallkammer nach der ersten Abfallkammer durch ein Öffnerelement mit dem ersten Kanalabschnitt fluidisch verbindbar ist. Die Abfallkammern sind bevorzugt in Strömungsrichtung eines Fluids hinter dem Fangelement angeordnet, wenn die Vorrichtung ein Fangelement aufweist.

Strukturträger

In einer Ausführungsform der Erfindung sind die Kanalabschnitte, Öffner- und Schließerelemente, der Fangbereich der mikrofluidischen Vorrichtung und weitere Elemente wie Abluftventile und Abfallkammern in mindestens einem Strukturträger angeordnet bzw. werden durch diesen gebildet.

Der Strukturträger weist bevorzugt ein Material ausgewählt aus der Gruppe aufweisend Polyethylen (PE), Polyethylenterephthalat (PET), Polymethylmethacrylat (PMMA), Cycloolefin-Copolymer (COC) und Polycarbonat (PC) auf. Besonders bevorzugt ist der Strukturträger als selbstklebende Folie aus einem dieser Materialien gefertigt. Das mikrofluidische System beinhaltet in dieser Ausführungsform zudem mindestens eine Abdeckung, welche den Strukturträger zumindest teilweise abdeckt.

Die erfindungsgemäße mikrofluidische Vorrichtung kann beispielsweise mit folgenden Herstellungsverfahren hergestellt werden:

- Laserstrukturierung der einzelnen Layer und Fügen durch z. B. Heißpresse, Klebefilme;
- Soft-Lithographie (Herstellung eines Masters (z. B. Fotolack (z. B. SU8) oder Trockenfilmresist (DFR)) und Abformen in z. B. Polydimethylsiloxan (PDMS) oder Polyurethan (PU));
- 3D Druck;
- Hot-Embossing;
- Lithographie mit Nass/Trockenätzverfahren;
- Mikrozerspanung (Bohren und Fräsen);
- Ultraschallbearbeitung;
- elektroerosive bzw. elektrochemische Bearbeitung;
- e-beam Bearbeitung; und
- Focused Ion Beam Bearbeitung.

Vorteilhafterweise werden die Öffner- und Schließerelemente, durch die die Steuerung der fluidischen Vorrichtung umgesetzt wird durch die Fluide, die als Prozessmedien im fluidischen System fungieren, aktiviert. Das heißt die fluidische Probe oder die Fluide, die als Prozessmedien in die Aufnahmebereiche der Kanalabschnitte eingebracht werden, steuern direkt die Aktivierung der Öffner- und Schließerelemente.

In einer weiteren Ausführungsform ist das Fangelement als Verbindungselement zwischen zwei Strukturträgern angeordnet.

In einer Ausführungsform der vorliegenden Erfindung weist die mikrofluidische Vorrichtung weiterhin eine oder mehrere Druck- und/oder Flussquellen auf, welche den bzw. die nötigen Fluidtransporte realisieren. Bevorzugt sind die Druck- und/oder Flussquellen direkt in die fluidische Vorrichtung integriert oder einfach mit dieser kombinierbar. Besonders bevorzugt sind die Druck- und/oder Flussquellen so ausgeführt, dass sie keine elektrische Energie zum Betrieb benötigen. Derartige Druck- und/oder Flussquellen sind dem Fachmann beispielsweise aus der DE102010015161 bekannt.

Vorrichtung zu Probenanalytik

In einer Ausführungsform weist die erfindungsgemäße fluidische Vorrichtung weiterhin eine Vorrichtung zur Probenanalytik auf, welche in Strömungsrichtung eines Fluids nach dem Fangbereich des ersten Kanalabschnitts angeordnet ist, und mit dem ersten Kanalabschnitt, vorzugsweise durch mindestens ein Öffnerelement, fluidisch verbunden ist. Die Vorrichtung zur Probenanalytik kann erfindungsgemäß über ein oder auch mehrere Öffnerelemente am ersten Kanalabschnitt angeordnet sein.

In einer bevorzugten Ausführungsform ist die Vorrichtung zur Probenanalytik eine Vorrichtung zum Nachweis von Analyten,

wobei die Vorrichtung zur Probenanalytik $j$ Reaktionskammern aufweist, wobei j e N und $j \geq 1$;
wobei jede Reaktionskammer einen Einlass und einen Auslass aufweist; und
wobei in Strömungsrichtung eines Fluids vor dem Einlass jeder Reaktionskammer ein Schließerelement angeordnet ist und am Auslass jeder Reaktionskammer ein Abluftventil und/oder Schließerelement angeordnet ist.

In einer weiteren Ausführungsform ist die Vorrichtung zur Probenanalytik eine Vorrichtung zur Entnahme des isolierten Analyten, um diesen dann anderen/extern durchzuführenden Analysen zuzuführen.

In einer Ausführungsform werden die nachzuweisenden Analyte nach Elution vom Fangelement in der Elutionslösung in die Vorrichtung zur Probenanalytik geleitet.

In einer Ausführungsform können in einer oder mehreren der $j$ Reaktionskammern getrocknete Reagenzien enthalten sein. Vorzugsweise sind die Reagenzien durch Hitzetrocknung oder Lyophilisieren konserviert.

In einer Ausführungsform sind die Reagenzien Reagenzien für einen isothermalen Nachweis von Nukleinsäuren. Dies können zum Beispiel Schleifen-vermittelte isothermale Amplifikation (LAMP), Rolling Circle Amplifikation (RCA), Rekombinase Polymerase Amplifikation (RPA) oder ähnliche sein. Hierdurch werden komplexe Temperaturprotokolle vermieden.

Das Schließerelement am Einlass jeder Reaktionskammer erlaubt die Dosierung des Eluats sowie ein "Abdichten" der Kammer (gegenüber benachbarter Reaktionskammern und der Zuführung), um Kreuzkontaminationen zu unterbinden. Die Schließerelemente am Einlass und Auslass jeder Reaktionskammer verschießen die jeweilige Reaktionsammer nach vollständiger Befüllung der Reaktionskammer vor der beginnenden biochemischen Nachweisreaktion, und verhindern so einen späteren Austritt von Reaktionslösungen aus der Reaktionskammer.

In einer Ausführungsform weist die Vorrichtung zur Probenanalytik weiterhin $l$ Abluftventile auf, wobei $l \in \mathbb{N}$ und $l \geq 1$ und $l \leq j$, wobei jeweils genau ein Abluftventil im Auslass einer Reaktionskammer angeordnet ist. Durch das Abluftventil wird ein Entweichen von Luft ermöglicht, während Fluide zurückgehalten werden. So kann ein Verbleiben von Luftblasen vor dem Verschließen der Reaktionskammern verhindert wird. Besonders bevorzugt sind die $l$ Abluftventile permeable Membranen.

In einer besonders bevorzugten Ausführungsform umfasst die Vorrichtung zur Probenanalytik zwei oder mehr Reaktionskammern, die derart angeordnet sind, dass eine gleichmäßige, parallele und gleichschnelle Befüllung der Reaktionskammern erfolgt. Vorteilhafterweise wird hierdurch ein gleichzeitiger Nachweis (multiplexer Nachweis) unterschiedlicher Analyte in einer Elutionslösung ermöglicht.

In einer weiteren Ausführungsform ist vor den $j$ Reaktionskammern eine Mischkammer angeordnet, die eine Durchmischung des Eluats vor dem Befüllen der $j$ Reaktionskammern ermöglicht, um einen möglichen Elutionsgradienten auszugleichen und so die Befüllung jeder der $j$ Reaktionskammern mit der gleichen Konzentration an Analyten erlaubt.

In einer weiteren Ausführungsform der vorliegenden Erfindung weist die erfindungsgemäße fluidische Vorrichtung weiterhin ein Temperierelement auf. Bevorzugt weist die Vorrichtung zur Probenanalytik ein Temperierelement auf, womit eine Temperierung der Fluide vorgenommen werden kann. Somit können Fluide derart temperiert werden, wie es für chemische Untersuchungen erforderlich ist. Das Temperierelement kann dabei in die fluidische Vorrichtung integriert sein oder mit dieser kombiniert werden. Bevorzugt wird ein elektrisches Temperierelement verwendet. Das Temperierelement kann aber auch derart ausgeführt sein, dass keine elektrische Energie zu dessen Betrieb benötigt wird.

In einer Ausführungsform weist die Vorrichtung zur Probenanalytik weiterhin mindestens einen Aufnahmebereich für ein Fluid auf. Durch den mindestens einen Aufnahmebereich der Probenanalytik können Prozessmedien der Vorrichtung zur Probeanalytik zugeführt werden. In einer weiteren Ausführungsform weist die Vorrichtung zur Probenanalytik weiterhin Kavitäten auf, die mit einem Aufnahmebereich der Vorrichtung zur Probenanalytik in Verbindung stehen. In dieser Ausführungsform können Proben in die Kavitäten pipettiert werden. Die gefüllten Kavitäten werden anschließend mechanisch verschlossen und/oder versiegelt. Reagenzien und Analyten können so in der Vorrichtung für die automatische Prozessabfolge vorgelagert werden. Die Anzahl der manuellen Schritte für eine Probenanalytik mit der erfindungsgemäßen Vorrichtung reduziert sich dann auf

1. die Probeneingabe in den Aufnahmebereich des ersten Kanalabschnittes, wenn alle weiteren Fluide (Prozessmedien) sich bereits in den Aufnahmebereichen der zweiten, dritten und/oder *k*-ten Kanalabschnitte und/oder der Vorrichtung zur Probenanalytik befinden oder
2. Probeneingabe in den Aufnahmebereich des ersten Kanalabschnittes und die Aktivierung der Vorrichtung durch das Einbringen der Fluide (Prozessmedien) in die Aufnahmebereiche der zweiten, dritten und/oder *k*-ten Kanalabschnitte und/oder die Vorrichtung zur Probenanalytik der fluidischen Vorrichtung.

Verfahren

Weiterhin stellt die Erfindung ein Verfahren zur sequentiellen Bereitstellung von definierten Fluidvolumina bereit mit einer fluidischen Vorrichtung aufweisend einen ersten Kanalabschnitt und mindestens einen zweiten Kanalabschnitt, mindestens ein Öffnerelement und mindestens ein Schließerelement
wobei

- der mindestens eine zweite Kanalabschnitt durch ein Öffnerelement angrenzend an den ersten Kanalabschnitt angeordnet ist und das Öffnerelement dazu eingerichtet ist, eine fluidische Verbindung von dem mindestens einen zweiten Kanalabschnitt zum ersten Kanalabschnitt herzustellen;
- der erste und der mindestens eine zweite Kanalabschnitt jeweils einen Aufnahmebereich aufweisen,
- im ersten Kanalabschnitt in Strömungsrichtung eines Fluids dem Öffnerelement ein Schließerelement vorgelagert angeordnet ist;

**umfassend die Schritte**

a) Bereitstellen eines ersten Fluids im Aufnahmebereich des ersten Kanalabschnitts, wodurch eine Strömungsrichtung des ersten Fluids in dem ersten Kanalabschnitt ausgebildet wird;
b) Bereitstellen eines zweiten Fluids in einem Aufnahmebereich eines mindestens einen zweiten Kanalabschnitts, wodurch eine Strömungsrichtung des zweiten Fluids in dem mindestens einen zweiten Kanalabschnitt ausgebildet wird;
c) Aktivieren des Schließerelementes durch das erste Fluid im ersten Kanalabschnitt und Strömen des ersten Fluids durch den ersten Kanalabschnitts bis das Schließerelement geschlossen ist;
d) Aktivieren des mindestens einen Öffnerelementes durch das erste Fluid im ersten Kanalabschnitt und/oder aktivieren des mindestens einen Öffnerelementes durch das zweite Fluid im mindestens einen zweiten Kanalabschnitt und Öffnen einer fluidischen Verbindung zwischen dem ersten und dem mindestens einen zweiten Kanalabschnitt;
e) Einströmen des zweiten Fluids aus dem mindestens einen zweiten Kanalabschnitt in den ersten Kanalabschnitt und Durchströmen des ersten Kanalabschnitt in Strömungsrichtung des ersten Fluids.

Alle Merkmale, die für die erfindungsgemäße Vorrichtung beschrieben wurden, treffen in gleicher Weise auf das erfindungsgemäße Verfahren zu und umgekehrt.
In einer bevorzugten Ausführungsform ist die erfindungsgemäße fluidische Vorrichtung dazu eingerichtet, das erfindungsgemäße Verfahren durchzuführen.
Verfahrensgemäß wird ein erstes Fluid im Aufnahmebereich des ersten Kanalabschnittes bereitgestellt. Das heißt, das erste Fluid wird in diesen eingebracht, wodurch eine Strömungsrichtung des ersten Fluids in dem ersten Kanalabschnitt ausgebildet wird. Das erste Fluid kann beispielsweise tierischen oder humanen Ursprungs sein oder auch eine Flüssigkeit mit zu analysierenden Bestandteilen. In einer Ausführungsform ist das erste Fluid ein Lyse- und Bindungspuffer mit zu analysierenden Bestandteilen.
Erfindungsgemäß wird im Aufnahmebereich mindestens eines zweiten Kanalabschnittes ein zweites Fluid bereitgestellt. Das heißt, es wird das zweite Fluid in diesen eingebracht, wodurch eine Strömungsrichtung des zweiten Fluids in dem mindestens einen zweiten Kanalabschnitt ausgebildet wird.

Das erste Fluid durchströmt den ersten Kanalabschnitt und aktiviert dabei das mindestens eine Schließerelement. Das erste Fluid strömt so lange durch den ersten Kanalabschnitt, bis das Schließerelement geschlossen ist. Hierdurch kann das Volumen des ersten Fluids welches durch den ersten Kanalabschnitt strömt exakt definiert werden.

Nach Kontakt des ersten Fluids mit dem mindestens einen Schließerelement wird das Öffnerelement durch Kontakt mit dem ersten Fluid im ersten Kanalabschnitt und/oder aktivieren des Öffnerelementes durch das zweite Fluid im mindestens einen zweiten Kanalabschnitt aktiviert. Ist das mindestens eine Öffnerelement geöffnet, ist damit eine fluidischen Verbindung zwischen dem ersten und dem mindestens einen zweiten Kanalabschnitt hergestellt.

Das zweite Fluid kann nun aus dem mindestens einen zweiten Kanalabschnitt in den ersten Kanalabschnitt einströmen und diesen in der gleichen Strömungsrichtung des ersten Fluids zuvor, durchströmen.

Durch die Aktivierung des Öffnerelementes kann der zeitliche Ablauf des Verfahrens genau definiert werden. Das heißt es kann sowohl zu einer definierten Zeit ein definiertes Volumen des ersten Fluids durch den ersten Kanalabschnitt geleitet werden als auch zu einer definierten Zeit ein definiertes Volumen des zweiten Fluids.

In einer Ausführungsform des erfindungsgemäßen Verfahrens weist die fluidische Vorrichtung weiterhin ein Fangelement im ersten Kanalabschnitt nach dem Öffnerelement auf, durch welche die Fluide hindurchströmen. Bei der Passage des Fangelementes wird zumindest ein Bestandteil des ersten Fluids zurückgehalten und immobilisiert.

In einer weiteren Ausführungsform des erfindungsgemäßen Verfahrens weist die fluidische Vorrichtung weiterhin eine Vorrichtung zur Probenanalytik auf, welche durch ein Öffnerelement am ersten Kanalabschnitt angeordnet ist, wobei das Öffnerelement dazu eingerichtet ist eine fluidische Verbindung vom ersten Kanalabschnitt zu der Vorrichtung zur Probenanalytik herzustellen;

wobei die Vorrichtung zur Probenanalytik in Strömungsrichtung eines Fluids hinter dem Öffnerelement angeordnet ist welches den zweiten mit dem ersten Kanalabschnitt verbindet oder hinter dem Fangelement im ersten Kanalabschnitt;

und wobei ein Fluidstrom im ersten Kanalabschnitt das Öffnerelement aktiviert, welches die Vorrichtung zur Probenanalytik mit dem ersten Kanalabschnitt fluidisch verbindet.

In einer weiteren Ausführungsform des Verfahrens zur sequentiellen Bereitstellung von definierten Fluidvolumina mit einer fluidischen Vorrichtung, weist die fluidische Vorrichtung

einen ersten Kanalabschnitt und n zweite Kanalabschnitte, wobei $n \in \mathbb{N}$ und $n \geq 1$, mindestens ein Öffnerelement und mindestens ein Schließerelement;
auf, wobei
der erste und die n zweiten Kanalabschnitte jeweils einen Aufnahmebereich aufweisen;

jeder der n zweiten Kanalabschnitte jeweils durch ein Öffnerelement an den ersten Kanalabschnitt angrenzt und jedes Öffnerelement jeweils dazu eingerichtet ist, eine fluidische Verbindung von einem zweiten Kanalabschnitt zum ersten Kanalabschnitt herzustellen;
jeweils ein Schließerelement vor jedem Öffnerelement in Strömungsrichtung eines Fluids im ersten Kanalabschnitt angeordnet ist;

aufweisend die Schritte

a. Bereitstellen eines ersten Fluids im Aufnahmebereich des ersten Kanalabschnittes, wodurch eine Strömungsrichtung des ersten Fluids in dem ersten Kanalabschnitt ausgebildet wird;
b. Durchströmen des ersten Fluids durch den ersten Kanalabschnitt, wobei das Schließerelement zwischen Aufnahmebereich des ersten Kanalabschnittes und dem ersten Öffnerelement in Flussrichtung des ersten Fluids durch das erste Fluid aktiviert wird;
c. Bereitstellen eines zweiten Fluids im Aufnahmebereich eines zweiten Kanalabschnittes, der an das erste Öffnerelement angrenzt, wodurch eine Strömungsrichtung des zweiten Fluids in dem zweiten Kanalabschnitt ausgebildet wird;
d. Aktivieren des ersten Öffnerelementes durch das zweite Fluid im zweiten Kanalabschnitt und/oder durch das erste Fluid im ersten Kanalabschnitt und Öffnen einer fluidischen Verbindung zwischen dem ersten und dem zweiten Kanalabschnitt;
e. Einströmen des zweiten Fluids aus dem zweiten Kanalabschnitt in den ersten Kanalabschnitt und Durchströmen des ersten Kanalabschnittes;
f. Aktivieren eines Schließerelementes welches zwischen dem zuletzt aktivierten Öffnerelement und einem weiteren in Strömungsrichtung des ersten Kanalabschnittes angeordneten Öffnerelementes angeordnet ist, bis dieses geschlossen ist durch das durch den ersten Kanalabschnitt strömende Fluid;

g. Bereitstellen eines weiteren Fluids im Aufnahmebereich eines weiteren zweiten Kanalabschnittes, der durch das weitere Öffnerelement mit dem ersten Kanalabschnitt fluidisch verbindbar ist;

h. Aktivieren des Öffnerelementes des weiteren zweiten Kanalabschnittes aus Verfahrensschritt g. durch das in Verfahrensschritt g. in den weiteren Kanalabschnitt eingebrachte Fluid und/oder durch das Fluid im ersten Kanalabschnitt;

i. Wiederholen der Schritte f. bis h. bis alle Öffnerelemente welche in Strömungsrichtung im ersten Kanalabschnitt vor dem Fangelement angeordnet sind, geöffnet sind.

Beispielsweise kann so ein Elutionspuffer und ein Waschpuffer zugeführt werden. Auf diese Weise können beliebig viele weitere Fluide dem ersten Kanalabschnitt mit einem definierten Volumen zeitlich definiert zugeführt werden.

In einer Ausführungsform werden dem ersten Kanalsystem 1, 2, 3, 4, oder 5 Fluide zugeführt.

In einer weiteren Ausführungsform weist die Vorrichtung weiterhin m dritte Kanalabschnitte auf. Erfindungsgemäß sind die m dritten Kanalabschnitte derart angeordnet, dass von diesen ein Fluid in einen zweiten Kanalabschnitt fließen kann und vom zweiten in den ersten Kanalabschnitt. Durch geeignete Wahl der Öffner- und Schließerelemente kann sowohl das Volumen der einzelnen Fluide als auch die zeitliche Abfolge der Verfahrensschritte exakt definiert werden.

In einer weiteren Ausführungsform des Verfahrens zur sequentiellen Bereitstellung von definierten Fluidvolumina mit einer fluidischen Vorrichtung, weist die fluidische Vorrichtung

einen ersten Kanalabschnitt, einen zweite Kanalabschnitt und $m$ dritte Kanalabschnitte, wobei $m \in \mathbb{N}$ und $m \geq 1$, mindestens ein Öffnerelement und mindestens ein Schließerelement;

auf, wobei

der erste, der zweite Kanalabschnitt und die $m$ dritten Kanalabschnitte jeweils einen Aufnahmebereich aufweisen;

der zweite Kanalabschnitt durch ein Öffnerelement an den ersten Kanalabschnitt angrenzt, wobei das Öffnerelement dazu eingerichtet ist, eine fluidische Verbindung vom zweiten Kanalabschnitt zum ersten Kanalabschnitt herzustellen;

jeder der m dritten Kanalabschnitte jeweils durch ein Öffnerelement an den zweiten Kanalabschnitt angrenzt und jedes Öffnerelement jeweils dazu eingerichtet ist, eine fluidische Verbindung von einem dritten Kanalabschnitt zum zweiten Kanalabschnitt herzustellen;

ein Schließerelement vor dem Öffnerelement in Strömungsrichtung eines Fluids im ersten Kanalabschnitt angeordnet ist;

aufweisend die Schritte

a. Bereitstellen eines ersten Fluids im Aufnahmebereich des ersten Kanalabschnittes, wodurch eine Strömungsrichtung des ersten Fluids in dem ersten Kanalabschnitt ausgebildet wird;

b. Durchströmen des ersten Fluids durch den ersten Kanalabschnitt, wobei das Schließerelement zwischen Aufnahmebereich des ersten Kanalabschnittes und dem ersten Öffnerelement in Flussrichtung des ersten Fluids durch das erste Fluid aktiviert wird;

c. Bereitstellen eines zweiten Fluids im Aufnahmebereich des zweiten Kanalabschnittes, der an das erste Öffnerelement angrenzt, wodurch eine Strömungsrichtung des zweiten Fluids in dem zweiten Kanalabschnitt ausgebildet wird;

d. Aktivieren des ersten Öffnerelementes welches den ersten Kanalabschnitt und den zweiten Kanalabschnitt verbindet durch das zweite Fluid im zweiten Kanalabschnitt und/oder durch das erste Fluid im ersten Kanalabschnitt und Öffnen einer fluidischen Verbindung zwischen dem ersten und dem zweiten Kanalabschnitt;

e. Einströmen des zweiten Fluids aus dem zweiten Kanalabschnitt in den ersten Kanalabschnitt und Durchströmen des ersten Kanalabschnittes;

f. Bereitstellen eines weiteren Fluids in einem Aufnahmebereich eines dritten Kanalabschnittes, wodurch eine Strömungsrichtung des Fluids in dem dritten Kanalabschnitt ausgebildet wird;

g. Aktivieren des Öffnerelementes welches den dritten Kanalabschnitt und den zweiten Kanalabschnitt verbindet durch das Fluid im zweiten Kanalabschnitt und/oder durch das Fluid im dritten Kanalabschnitt und Öffnen einer fluidischen Verbindung zwischen dem dritten und dem zweiten Kanalabschnitt;

h. Einströmen des Fluids aus dem dritten Kanalabschnitt in den zweiten Kanalabschnitt und vom zweiten Kanalabschnitt in den ersten Kanalabschnitt und Durchströmen des ersten Kanalabschnittes;

i. Bereitstellen eines weiteren Fluids im Aufnahmebereich eines weiteren dritten Kanalabschnittes, der durch ein weiteres Öffnerelement mit dem zweiten Kanalabschnitt fluidisch verbindbar ist;

j. Aktivieren des Öffnerelementes des weiteren dritten Kanalabschnittes aus Verfahrensschritt i. durch das in Verfahrensschritt i. in den weiteren dritten Kanalabschnitt eingebrachte Fluid und/oder durch das Fluid im zweiten Kanalabschnitt;

k. Einströmen des Fluids aus dem weiteren dritten Kanalabschnitt in den zweiten Kanalabschnitt und vom zweiten Kanalabschnitt in den ersten Kanalabschnitt und Durchströmen des ersten Kanalabschnittes;

l. Wiederholen der Schritte i. bis k. bis alle Öffnerelemente, welche einen dritten Kanalabschnitt mit dem zweiten Kanalabschnitt fluidisch verbinden, geöffnet sind.

In einer weiteren Ausführungsform weist die Vorrichtung nicht nur *m* dritte Kanalabschnitte sondern auch *f k*-te Kanalabschnitte auf. Erfindungsgemäß sind die *f k*-ten Kanalabschnitte jeweils durch ein Öffnerelement angrenzend an zumindest einen (*k-1*)-ten Kanalabschnitte angeordnet. Ein Fluid kann daher von einem *k*-ten Kanalabschnitt über einen (*k-1*)-ten Kanalabschnitt geleitet werden, bis es in einen dritten Kanalabschnitt und von diesem in einen zweiten und anschließend in den ersten Kanalabschnitt gelangt.

In einer weiteren Ausführungsform des Verfahrens zur sequentiellen Bereitstellung von definierten Fluidvolumina mit einer fluidischen Vorrichtung, weist die fluidische Vorrichtung

einen ersten Kanalabschnitt, einen zweite Kanalabschnitt, einen dritten und *f k-te* Kanalabschnitte, wobei $f \in \mathbb{N}$ und $f \geq 1$ und $k \in \mathbb{N}$ und $k \geq 4$, mindestens ein Öffnerelement und mindestens ein Schließerelement; auf, wobei

der erste, der zweite Kanalabschnitt, der dritte und die *f k-ten* Kanalabschnitte jeweils einen Aufnahmebereich aufweisen;

der zweite Kanalabschnitt durch ein Öffnerelement an den ersten Kanalabschnitt angrenzt, wobei das Öffnerelement dazu eingerichtet ist, eine fluidische Verbindung vom zweiten Kanalabschnitt zum ersten Kanalabschnitt herzustellen;

der dritte Kanalabschnitt durch ein Öffnerelement an den zweiten Kanalabschnitt angrenzt, wobei das Öffnerelement dazu eingerichtet ist, eine fluidische Verbindung vom dritten Kanalabschnitt zum zweiten Kanalabschnitt herzustellen;

jeder der *f k-ten* Kanalabschnitte durch ein Öffnerelement an einen (*k-1*)-ten Kanalabschnitt angrenzt und jedes Öffnerelement jeweils dazu eingerichtet ist, eine fluidische Verbindung von einem *k-ten* Kanalabschnitt zum (*k-1*)-ten Kanalabschnitt herzustellen,

jeder der *f k-ten* Kanalabschnitte, für k=4 jeweils durch ein Öffnerelement an den dritten Kanalabschnitt angrenzt und jedes Öffnerelement dazu eingerichtet ist eine fluidische Verbindung vom k-ten Kanalabschnitt zum dritten Kanalabschnitt herzustellen;

ein Schließerelement vor dem Öffnerelement in Strömungsrichtung eines Fluids im ersten Kanalabschnitt angeordnet ist;

aufweisend die Schritte

a. Bereitstellen eines ersten Fluids im Aufnahmebereich des ersten Kanalabschnittes, wodurch eine Strömungsrichtung des ersten Fluids in dem ersten Kanalabschnitt ausgebildet wird;

b. Durchströmen des ersten Fluids durch den ersten Kanalabschnitt, wobei das Schließerelement zwischen Aufnahmebereich des ersten Kanalabschnittes und dem ersten Öffnerelement in Flussrichtung des ersten Fluids durch das erste Fluid aktiviert wird;

c. Bereitstellen eines zweiten Fluids im Aufnahmebereich des zweiten Kanalabschnittes, der an das erste Öffnerelement angrenzt, wodurch eine Strömungsrichtung des zweiten Fluids in dem zweiten Kanalabschnitt ausgebildet wird;

d. Aktivieren des ersten Öffnerelementes welches den ersten Kanalabschnitt und den zweiten Kanalabschnitt verbindet durch das zweite Fluid im zweiten Kanalabschnitt und/oder durch das erste Fluid im ersten Kanalabschnitt und Öffnen einer fluidischen Verbindung zwischen dem ersten und dem zweiten Kanalabschnitt;

e. Einströmen des zweiten Fluids aus dem zweiten Kanalabschnitt in den ersten Kanalabschnitt und Durchströmen des ersten Kanalabschnittes;

f. Bereitstellen eines Fluids im Aufnahmebereich des dritten Kanalabschnittes, wodurch eine Strömungsrichtung des Fluids in dem dritten Kanalabschnitt ausgebildet wird;

g. Aktivieren des Öffnerelementes welches den dritten Kanalabschnitt und den zweiten Kanalabschnitt verbindet durch das Fluid im zweiten Kanalabschnitt und/oder durch das Fluid im dritten Kanalabschnitt und Öffnen einer fluidischen Verbindung zwischen dem dritten und dem zweiten Kanalabschnitt;

h. Einströmen des Fluids aus dem dritten Kanalabschnitt in den zweiten Kanalabschnitt und vom zweiten Kanalabschnitt in den ersten Kanalabschnitt und Durchströmen des ersten Kanalabschnittes;

i. Bereitstellen eines Fluids im Aufnahmebereich eines *k-ten* Kanalabschnittes, wodurch eine Strömungsrichtung des Fluids in dem *k-ten* Kanalabschnitt ausgebildet wird;

j. Aktivieren des Öffnerelementes welches den *k-ten* Kanalabschnitt und den *(k-1)-ten* Kanalabschnitt verbindet durch das Fluid im *k-ten* Kanalabschnitt und/oder durch das Fluid im *(k-1)-ten* Kanalabschnitt und Öffnen einer fluidischen Verbindung zwischen dem *k-ten* und dem *(k-1)-ten* Kanalabschnitt;

k. Einströmen des Fluids aus dem *k-ten* Kanalabschnitt in den *(k-1)-ten* Kanalabschnitt und Durchströmen der Kanalabschnitte bis zum ersten Kanalabschnitt;

l. Bereitstellen eines weiteren Fluids im Aufnahmebereich eines weiteren *k-ten* Kanalabschnittes, wodurch eine Strömungsrichtung des Fluids in dem weiteren k-ten Kanalabschnitt ausgebildet wird;

m. Aktivieren des Öffnerelementes welches den weiteren *k-ten* Kanalabschnitt und den *(k-1)-ten* Kanalabschnitt verbindet durch das Fluid im *k-ten* Kanalabschnitt und/oder durch das Fluid im *(k-1)-ten* Kanalabschnitt und Öffnen einer fluidischen Verbindung zwischen dem *k-ten* und dem *(k-1)-ten* Kanalabschnitt;

n. Einströmen des Fluids aus dem *k-ten* Kanalabschnitt in den *(k-1)-ten* Kanalabschnitt und Durchströmen der Kanalabschnitte bis zum ersten Kanalabschnitt;

o. Wiederholen der Schritte l. bis n. bis alle Öffnerelemente welche einen *k-ten* Kanalabschnitt mit einem *(k-1)-ten* Kanalabschnitt fluidisch verbinden geöffnet sind.

In einer weiteren Ausführungsform weist die fluidische Vorrichtung weiterhin ein Fangelement, eine Vorrichtung zur Probenanalytik und eine Abfallkammer mit einem Abluftventil auf.

In einer weiteren Ausführungsform des Verfahrens zur sequentiellen Bereitstellung von definierten Fluidvolumina mit einer fluidischen Vorrichtung, weist die fluidische Vorrichtung

einen ersten Kanalabschnitt und *n* zweite Kanalabschnitte, wobei $n \in \mathbb{N}$ und $n \geq 1$, mindestens ein Öffnerelement und mindestens ein Schließerelement;
ein Fangelement;
eine Vorrichtung zur Probenanalytik; und
eine Abfallkammer mit einem Abluftventil;
auf, wobei
der erste und die *n* zweiten Kanalabschnitte jeweils einen Aufnahmebereich aufweisen;

jeder der *n* zweiten Kanalabschnitte jeweils durch ein Öffnerelement an den ersten Kanalabschnitt angrenzt und jedes Öffnerelement jeweils dazu eingerichtet ist, eine fluidische Verbindung von einem zweiten Kanalabschnitt zum ersten Kanalabschnitt herzustellen;

jeweils ein Schließerelement vor jedem Öffnerelement in Strömungsrichtung eines Fluids im ersten Kanalabschnitt angeordnet ist;

das Fangelement nach dem letzten Öffnerelement in Strömungsrichtung eines Fluids im ersten Kanalabschnitt angeordnet ist;

die Vorrichtung zur Probenanalytik in Strömungsrichtung eines Fluids nach dem Fangelement im ersten Kanalabschnitt angeordnet ist und mit diesem durch mindestens ein Öffnerelement fluidisch verbindbar ist; und

der erste Kanalabschnitt in die Abfallkammer mündet, wobei am Eingang der Abfallkammer im ersten Kanalabschnitt ein Schließerelement angeordnet ist;

aufweisend die Schritte

a. Bereitstellen eines ersten Fluids im Aufnahmebereich des ersten Kanalabschnittes, wodurch eine Strömungsrichtung des ersten Fluids in dem ersten Kanalabschnitt ausgebildet wird;

b. Durchströmen des ersten Fluids durch den ersten Kanalabschnitt und das darin angeordnete Fangelement zur Abfallkammer, wobei das Schließerelement zwischen Aufnahmebereich des ersten Kanalabschnittes und dem ersten Öffnerelement in Flussrichtung des ersten Fluids durch das erste Fluid aktiviert wird und das Schließerelement vor der Abfallkammer;

c. Bereitstellen eines zweiten Fluids im Aufnahmebereich eines zweiten Kanalabschnittes, der an das erste Öffnerelement angrenzt, wodurch eine Strömungsrichtung des zweiten Fluids in dem zweiten Kanalabschnitt ausgebildet wird;

d. Aktivieren des ersten Öffnerelementes durch das zweite Fluid im zweiten Kanalabschnitt und/oder durch das erste Fluid im ersten Kanalabschnitt und Öffnen einer fluidischen Verbindung zwischen dem ersten und dem zweiten Kanalabschnitt;

e. Einströmen des zweiten Fluids aus dem zweiten Kanalabschnitt in den ersten Kanalabschnitt und Durchströmen des ersten Kanalabschnittes sowie des darin angeordneten Fangelementes;

f. Aktivieren mindestens eines Öffnerelementes, welches die Vorrichtung zur Probenanalytik mit dem ersten Kanalabschnitt fluidisch verbindet durch das durch den ersten Kanalabschnitt strömende Fluid;

g. Einströmen des Fluids aus dem ersten Kanalabschnitt in die Vorrichtung zur Probenanalytik.

Verfahrensgemäß wird ein erstes Fluid im Aufnahmebereich des ersten Kanalabschnittes bereitgestellt. Das heißt, das erste Fluid wird in diesen eingebracht, wodurch eine Strömungsrichtung des ersten Fluids in dem ersten Kanalabschnitt ausgebildet wird. Das erste Fluid durchströmt den ersten Kanalabschnitt und das darin angeordnete Fangelement bis zur Abfallkammer. Bei der Passage des Fangelementes wird zumindest ein Bestandteil des ersten Fluids zurückgehalten und immobilisiert. Durch das erste Fluid wird das Schließerelement zwischen Aufnahmebereich des ersten Kanalabschnittes und dem ersten Öffnerelement in Flussrichtung des ersten Fluids aktiviert. Hierdurch wird der Fluss des ersten Fluids vom Aufnahmebereich durch den ersten Kanalabschnitt blockiert, wodurch kein Fluid vom Aufnahmebereich zum Fangelement mehr nachfließt und damit eine Dosierung des ersten Fluids vorgenommen wird. Weiterhin wird das Schließerelement vor der Abfallkammer aktiviert, so dass dieses nach einer definierten Zeit geschlossen ist.

In den Aufnahmebereich eines zweiten Kanalabschnittes, der an das erste Öffnerelement angrenzt, wird ein zweites Fluid eingebracht. Das erste Öffnerelement wird erfindungsgemäß durch das zweite Fluid im zweiten Kanalabschnitt und/oder durch das erste Fluid im ersten Kanalabschnitt aktiviert, so dass nach einer definierten Zeit eine fluidische Verbindung zwischen dem ersten und dem zweiten Kanalabschnitt besteht. Hierdurch gelangt das zweite Fluid aus dem zweiten Kanalabschnitt in den ersten Kanalabschnitt, durchströmt diesen in Richtung des Fangelementes und durchströmt das Fangelement. Erfindungsgemäß aktiviert das Fluid im ersten Kanalabschnitt mindestens ein Öffnerelement, welches die Vorrichtung zur Probenanalytik mit dem ersten Kanalabschnitt fluidisch verbindet und strömt, wenn das mindestens eine Öffnerelement geöffnet ist aus dem ersten Kanalabschnitt in die Vorrichtung zur Probenanalytik. Erfindungsgemäß wird die zeitliche Abfolge des Verfahrens durch das Aktivieren der Öffner- und Schließerelemente bestimmt. Insbesondere auch durch die konstruktive Anordnung der Elemente der Vorrichtung und deren.

Das Fluid, welches im Verfahrensschritt c. eingebracht wird, kann beispielsweise ein Waschpuffer sein.

Das erfindungsgemäße Verfahren ist insbesondere vorteilhaft zur zeitlichen Steuerung einer sequentiellen Prozessabfolge von zumindest zwei Flüssigkeiten in einer fluidischen Vorrichtung. Durch geeignete Wahl der Parameter, wie Öffner- und Schließerelemente kann prozessabhängig die jeweils gewünschte zeitliche Abfolge von Prozessschritten, wie Dosierung, Immobilisierung, Waschen, Eluieren, Auflösen von Barrieren sowie Verschluss gewünschter Kanalabschnitte mittels Schließerelemente erzielt werden.

In einer Ausführungsform der vorliegenden Erfindung ist nach dem ersten Öffnerelement und vor dem Fangbereich im ersten Kanalabschnitt ein weiteres Schließerelement angeordnet. Dieses wird bevorzugt durch das Fluid aktiviert, welches durch den weiteren Kanalabschnitt in Schritt c. eingebracht wird. Das Schließerelement verschließt den ersten Kanalabschnitt vorzugsweise, wenn ein definiertes Volumen des Fluids hindurchgeströmt ist. Diese Ausführungsform dient der Volumendefinition des Fluids.

In einer weiteren Ausführungsform weist das erfindungsgemäße Verfahren nach dem zuletzt beschriebenen Verfahrensschritt e. und vor dem zuletzt beschriebenen Verfahrensschritt f. weiterhin die Schritte

ea. Aktivieren eines Schließerelementes welches zwischen dem zuletzt aktivierten Öffnerelement und einem weiteren in Strömungsrichtung des ersten Kanalabschnittes angeordneten Öffnerelementes angeordnet ist, bis dieses geschlossen ist durch das durch den ersten Kanalabschnitt strömende Fluid;

eb. Bereitstellen eines weiteren Fluids im Aufnahmebereich eines weiteren zweiten Kanalabschnittes, der durch das weitere Öffnerelement mit dem ersten Kanalabschnitt fluidisch verbindbar ist;

ec. Aktivieren des Öffnerelementes des weiteren zweiten Kanalabschnittes aus Verfahrensschritt eb. durch das in Verfahrensschritt eb. in den weiteren Kanalabschnitt eingebrachte Fluid und/oder durch das Fluid im ersten Kanalabschnitt;

ed. Wiederholen der Schritte ea. bis ec. bis alle Öffnerelement welche in Strömungsrichtung im ersten Kanalabschnitt vor dem Fangelement angeordnet sind geöffnet sind

auf.

In einer weiteren Ausführungsform weist die Vorrichtung weiterhin *m* dritte Kanalabschnitte auf. Erfindungsgemäß sind die *m* dritten Kanalabschnitte wie bereits beschrieben derart angeordnet, dass von diesen ein Fluid in einen zweiten Kanalabschnitt fließen kann und vom zweiten in den ersten Kanalabschnitt. Durch geeignete Wahl der Öffner- und Schließerelemente kann sowohl das Volumen der einzelnen Fluide als auch die zeitliche Abfolge der Verfahrensschritte exakt definiert werden.

In einer weiteren Ausführungsform weist die Vorrichtung nicht nur *m* dritte Kanalabschnitte sondern auch *f k*-te Kanalabschnitte auf. Erfindungsgemäß sind die *f k*-ten Kanalabschnitte jeweils durch ein Öffnerelement angrenzend an zumindest einen (*k-1*)-ten Kanalabschnitte angeordnet. Ein Fluid kann daher wie bereits beschrieben von einem *k*-ten Kanalabschnitt über einen (*k-1*)-ten Kanalabschnitt geleitet werden, bis es in einen dritten Kanalabschnitt und von diesem in einen zweiten und anschließend in den ersten Kanalabschnitt gelangt.

Weist die Vorrichtung m dritte Kanalabschnitte oder auch zusätzlich *f k*-te Kanalabschnitte auf, so können in gleicher Weise wie bereits beschrieben in der Vorrichtung ebenfalls ein Fangelement, eine Vorrichtung zur Probenanalytik und eine Abfallkammer mit einem Abluftventil auf.

Das Öffnerelement, welches den ersten Kanalabschnitt mit der Vorrichtung zur Probenanalytik verbindet, wird in einer Ausführungsform bereit durch den Kontakt mit dem Fluid, welches in den Aufnahmebereich des ersten Kanalabschnittes eingebracht wird, aktiviert, d.h. die Aktivierung des Öffnerelementes wird initiiert. Die Aktivierungszeit ist dann an den Verfahrensablauf angepasst. In einer Ausführungsform kann daher die Aktivierung dieses Öffnerelementes durch das Fluid, welches in den Aufnahmebereich des ersten Kanalabschnittes eingebracht wird, initiiert werden und die Aktivierungszeit ist abgeschlossen, wenn das letzte Fluid aus einem zweiten, dritten oder *k*-ten Kanalabschnitt durch den ersten Kanalabschnitt strömt. Dieses kann dann durch das geöffnete Öffnerventil in die Vorrichtung zur Probenanalytik einströmen.

Die vorliegende Erfindung kann als chemofluidisches System bzw. chemofluidische Vorrichtung zur Vorbereitung von Proben für weitere Analysen zum Beispiel auf Basis von Nukleinsäuren, Proteinen, Lipiden und/oder Kohlenhydraten verwendet werden. Analysen können Prozesse auf Basis von Polymerase-Kettenreaktion, isotherme Amplifikation, Detektion von Enzymaktivitäten und/oder weiterer biochemischer Nachweisreaktionen umfassen.

Die Vorrichtung zur Probenanalytik kann erfindungsgemäß vielfältig ausgestaltet sein und wird entsprechend des jeweiligen Anwendungsfalls ausgestaltet. Die Vorrichtung zur Probenanalytik weist in einer Ausführungsform Öffnerelemente und/oder Schließerelemente entsprechend der vorliegenden Erfindung auf.

Ein großer Vorteil der vorliegenden Erfindung liegt darin, dass durch Integration eines Fangelementes in ein chemofluidisches System, zumindest ein Bestandteil einer Probe unter geschickter Kombination von Öffnerelementen und Schließerelementen zurückgehalten/immobilisiert werden kann. Somit erlaubt die erfindungsgemäße Vorrichtung als chemofluidisches System durch hilfsenergiefreie Automatisierung der komplexen Prozessabfolge die Vorbereitung einer Probe tierischen oder humanen Ursprungs für eine nachfolgende Analyse mittels z. B. isothermer Amplifikation.

Die vorliegende Erfindung ermöglicht eine automatisierte und hilfsenergiefreie Durchführung komplexer biochemischer Prozessabfolgen, wie sie häufig bei der Probenvorbereitung von biologischen Proben für die nachfolgende Analytik anzutreffen sind. Durch die Kombination von Schließer- und Öffnerelementen wird eine Steuerung der Prozessabfolge in dem mikrofluidischen System- aktiviert durch die Prozessmedien - realisiert und eine externe Steuerung wird unnötig. Dies bringt zudem Vorteile im Bereich der Parallelisierung und Miniaturisierung. Bei den Schließerelementen können Elemente ab $50\,\mu m$ erfolgreich photostrukturiert werden, bei den Öffnerelementen ist dies ab $125\,\mu m$ Lochdurchmesser (Lochdesign) möglich. Eine Skalierung der Kanalabschnitte in diesen Größenordnungen ist möglich.

Durch die Integration eines Fangelementes können gezielt Bestandteile einer Probe zurückgehalten/immobilisiert werden, um sie einer nachfolgenden Analyse zugänglich zu machen. Durch die Immobilisierung und sich an die Anbindung anschließende Waschschritte können Bestandteile der Probe, die störend auf die Nachweisreaktionen wirken, entfernt werden, ohne die zu analysierenden Bestandteile der Probe zu verlieren.

In einer weitere Ausführungsform weist der erste Kanal der vorliegenden Erfindung eine Mischerstruktur als Aufnahmebereich auf. In einer der Mischerstruktur vorgelagerten Kanalstruktur wird über einen Aufnahmebereich eine fluidische Probe, z.B. eine Patientenprobe aufgegeben sowie über einen weiteren Aufnahmebereich eine weitere fluidische Probe, z.B. ein Lyse/Bindepuffer. Die beiden fluidischen Proben werden parallel in die Mischerstruktur geleitet, die als Aufnahmebereich des ersten Kanalabschnitts fungiert. Die Volumen beider fluidischen Proben sind vordefiniert und in der Mischerstruktur erfolgt die Vermischung der beiden fluidischen Proben. Diese Mischung strömt weiter durch den ersten Kanalabschnitt über ein Fangelement, welches eine permeable Membran sein kann, in eine Abfallkammer. Durch ein in der Abfallkammer platziertes Abluftventil kann die in der Kanalstruktur vorhandene Luft entweichen, wobei keine Flüssigkeit austreten kann. Die Mischung aus fluidischer Probe und weiteren fluidischen Probe aktiviert auf ihrem Weg von der Mischerstruktur zur Abfallkammer Öffnerelemente und Schließerelemente.

Über einen Aufnahmebereich eines zweiten Kanalabschnittes wird ein erster Puffer, z. B. ein Waschpuffer, auf den Kanalstrukturträger gegeben. Der Puffer fließt durch den zweiten Kanalabschnitt zu einer permeablen Membran, welche Luft, aber keine Flüssigkeit aus der Kanalstruktur entweichen lässt. Die permeable Membran ist in dieser Ausführungsform Teil eines Abluftventils im zweiten Kanalabschnitts. Der Puffer aktiviert beim Durchströmen des zweiten Kanalabschnitts ein an den ersten Kanalabschnitt angrenzendes Öffnerelement. Nach einer definierten Zeit öffnet dieses Öffnerelement, so dass der Puffer vom zweiten Kanalabschnitt in den ersten Kanalabschnitt in Richtung des Fangelementes fließen kann. Der Puffer strömt über das Fangelement und kann nicht immobilisierte Bestandteile vom Fangelement entfernen. Das Volumen des zweiten Puffers wird in dieser Ausführungsform über die Größe der Abfallkammer hinter dem Fangelement im ersten Kanalabschnitt definiert. Ist die Abfallkammer mit den gemischten fluidischen Proben und dem Puffer gefüllt, stoppt der Zufluss an Puffer.

Über einen Aufnahmebereich in einem weiteren zweiten Kanalabschnitt kann ein zweiter Puffer, z. B. ein Elutionspuffer, auf den Kanalstrukturträger aufgegeben werden. Dieser fließt in dem weiteren zweiten Kanalabschnitt zu einer permeablen Membran, welche Luft, aber keine Flüssigkeit aus der Kanalstruktur entweichen lässt. Die permeable Membran ist in dieser Ausführungsform Teil eines Abluftventils im zweiten Kanalabschnitt. Der zweite Puffer aktiviert beim Durchströmen des weiteren zweiten Kanalabschnitts ein an den ersten Kanalabschnitt angrenzendes Öffnerelement. Nachdem der Zufluss des ersten Puffers, z. B. Waschpuffers, gestoppt ist, öffnet das Öffnerelement zwischen dem ersten Kanalabschnitt und dem weiteren zweiten Kanalabschnitt und lässt den zweiten Puffer vom weiteren zweiten Kanalabschnitt in den ersten Kanalabschnitt in Richtung der Reaktionskammern fließen, die zu einer Vorrichtung zur Probenanalytik gehören. Der zweite Puffer strömt dabei ebenfalls über das Fangelement und kann die dort immobilisierten Bestandteile der fluiden Probe ablösen.

Die Reaktionskammern, die zur Vorrichtung zur Probenanalytik, gehören, sind durch ein Öffnerelement mit dem ersten Kanalabschnitt verbunden. Wenn dieses Öffnerelement, nachdem der Zufluss des zweiten Puffers, z. B. Elutionspuffer, öffnet, wird der Strömungsweg für den zweiten Puffer, z. B. Elutionspuffer mit vom Fangelement abgelösten Bestandteilen, in die Reaktionskammern, die zur Vorrichtung zur Probenanalytik gehört, freigegeben. Der Puffer kann in Richtung der Reaktionskammern strömen, wobei jeweils ein Schließerelement vor jeder Reaktionskammer und nach jeder Reaktionskammer angeordnet ist, um das Volumen der Flüssigkeit in der Reaktionskammer zu definieren und die Strömungswege sofort zu schließen. Die Schließerelemente werden dabei von nachgelagerten permeablen Membranen unterstützt und erlauben lediglich das Entweichen von Luft und nicht von Flüssigkeit.

Im Folgenden wird die vorliegende Erfindung weiterhin anhand von 15 Figuren und 4 Ausführungsbeispielen näher erläutert.

Figur 1     (A) und (B) stellen ein Öffnerelement dar;
Figur 2     stellt eine Ausführung des Öffnerelements dar;
Figur 3     (A) und (B) stellen ein Schließerelement dar;
Figur 4     (A) und (B) stellen Fangelemente dar;
Figur 5     (A) bis (D) stellt Ausführungsformen des Fangelementes dar;
Figur 6     stellt eine Ausführungsform der erfindungsgemäßen Vorrichtung dar;
Figur 7     (A) und (B) stellen Ausführungsformen der vorliegenden Erfindung dar;
Figur 8     (A) und (B) stellen Ausführungsformen der vorliegenden Erfindung dar;
Figur 9     stellt eine Ausführungsform der vorliegenden Erfindung dar;
Figur 10    (A) und (B) stellen eine Ausführungsform der vorliegenden Erfindung dar;
Figur 11    stellt eine Ausführungsform der vorliegenden Erfindung dar;
Figur 12    stellt eine Ausführungsform der vorliegenden Erfindung dar;
Figur 13    (A) und (B) stellen Öffnungszeiten für verschiedene Öffnerelemente dar;
Figur 14    (A) und (B) stellen Schließzeiten für verschiedene Schließerelemente dar;
Figur 15    Ausführungsformen für Mischerstrukturen/Aufnahmebereich.

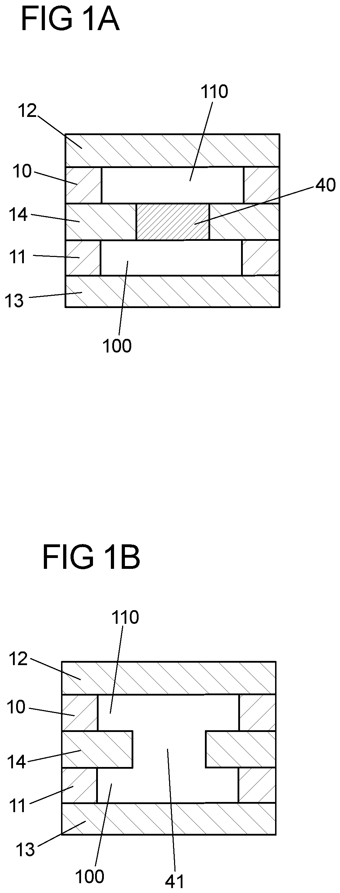

**Figur 1 (A) und (B)** stellen den prinzipiellen Aufbau und die Funktionsweise eines zeit- und ereignisgesteuerten Öffnerelementes 40 dar. Figur 1 (A) stellt das Öffnerelement (40) im geschlossenen Zustand dar und Figur 1 (B) im geöffneten Zustand.

Das Öffnerelement 40 trennt einen ersten Kanalabschnitt 100 im Strukturträger 11 und einen zweiten Kanalabschnitt 110 im Strukturträger 10. Wenn ein Prozessmedium durch einen der Kanalabschnitte 100, 110 auf das Öffnerelement 40 in Strukturträger 14 trifft, initiiert es dessen Auflösevorgang. Nach einer definierten Zeit (durch Design, Material etc. bestimmt) ist das Material des Öffnerelementes 40 komplett aufgelöst und eine Verbindung 41 zwischen dem ersten Kanalabschnitt 100 und dem zweiten Kanalabschnitt 110 ist geschaffen. Die Strukturträger 12 und 13 bilden jeweils eine Abdeckung.

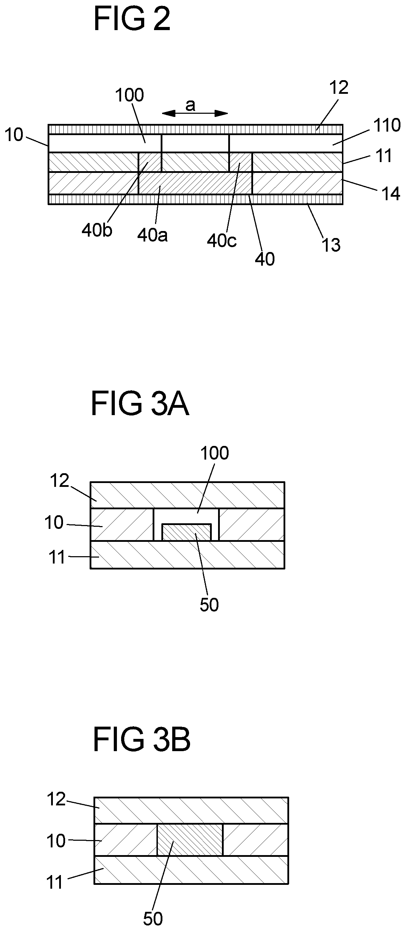

**Figur 2** stellt eine weitere Ausführungsform des Öffnerelementes 40 dar. In dieser Ausführungsform sind ein ersten Kanalabschnitt 100 und ein zweiten Kanalabschnitt 110 peripher zueinander angeordnet. Das Öffnerelement 40 ist in den Strukturträgern 11 und 14, die an den Strukturträger 10, welcher die Kanalabschnitte 100 und 110 aufweist, angeordnet. Das Öffnerelement 40 überlappt dabei mit dem Kanalabschnitt 100 und dem Kanalabschnitt 110. Das Öffnerelement 40 weist einen kanalförmigen Teil 40 a sowie zwei Verbindungselemente 40b, 40c auf. Wenn ein Prozessmedium durch einen der Kanalabschnitte 100, 110 oder durch beide Kanalabschnitte auf das Öffnerelement 40 trifft, initiiert es dessen Auflösevorgang. Nach einer definierten Zeit (durch Design, Material etc. bestimmt) ist das Material des Öffnerelementes 40 komplett aufgelöst und eine Verbindung zwischen dem ersten Kanalabschnitt 100 und dem zweiten Kanalabschnitt 110 ist geschaffen. Die Stufenbreite es Öffnerelementes 40 ist mit dem Bezugszeichen a gekennzeichnet.

**Figur 3 (A) und (B)** stellen den prinzipiellen Aufbau und die Funktionsweise eines zeit- und ereignisgesteuerten Schließerelementes 50 dar. Das Schließerelement ist auf dem Strukturträger 11 aufgebracht und der Kanalabschnitt 100 in den Strukturträger 10. Der Strukturträger 12 bildet eine Abdeckung. Die Figur stellt beispielhaft ein Schließerelement dar, welches zunächst ereignis-, dann zeitgesteuert betätigt wird. Die Aufgabe des Schließerelementes besteht darin, den Kanalabschnitt 100 nach Befüllen mit Prozessmedium zu verschließen, wobei durch den Schließvorgang das Volumen des Prozessmediums definiert wird. Wenn ein Prozessmedium durch den Kanalabschnitt 100 auf das Schließerelement 50 im getrockneten Zustand trifft (Figur 2(A)), initiiert es dessen Quellvorgang. Nach einer definierten Zeit (durch Design, Material etc. bestimmt) ist das Schließerelement 50 komplett gequollen und verschließt den Kanalabschnitt 100 (Figur 3 (B)).

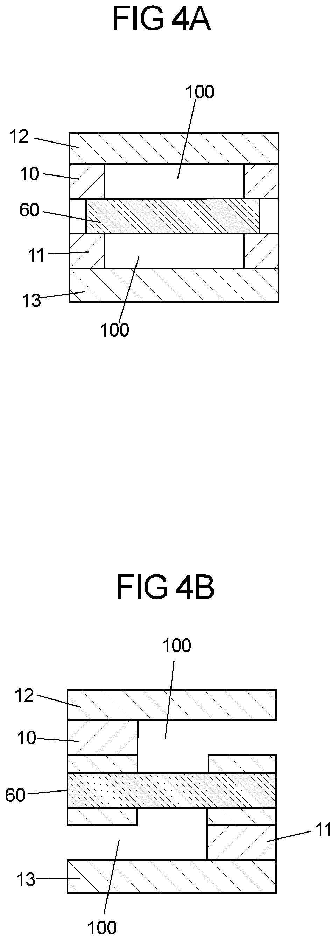

**Figur 4 (A) und (B)** stellen den prinzipiellen Aufbau von Fangelementen 60 dar. Figur 4 (A) stellt einen Querschnitt durch einen Kanalabschnitt 100 mit einer permeablen Membran als Fangelement 60 dar. Figur 4 (B) stellt einen Querschnitt in Längsrichtung durch einen Kanalabschnitt 100 mit einer permeablen Membran als Fangelement 60 dar.

In diesen Figuren ist beispielhaft die Integration eines Strukturträgers, der eine permeable Membran als Fangelement 60 umfasst, dargestellt. Das Fangelement 60 ist zwischen zwei Layern 10, 11 eingespannt, die im Bereich des Kanalabschnittes 100 einen direkten Kontakt mit der permeablen Membran des Fangelementes 60 erlauben. Das Fangelement 60 kann dabei als eigener Strukturträger aufgefasst werden. In einer Ausführungsform ist das Fangelement in Form einer Folie ausgeführt. In diese wird ein Loch gelasert, in welches eine Cellulose- oder Silica-Membran eingesetzt wird. So strömt/fließt ein Prozessmedium von einem Teil des Kanalabschnittes 100, der in einem ersten Strukturträger 10 angeordnet ist, durch das Fangelement 60 in einen weiteren Teil des Kanalabschnittes 100, der in einem zweiten Strukturträger 11 angeordnet ist. Dabei werden durch die permeable Membran des Fangelementes 60 Bestandteile des Prozessmediums bzw. der Probe zurückgehalten und immobilisiert.

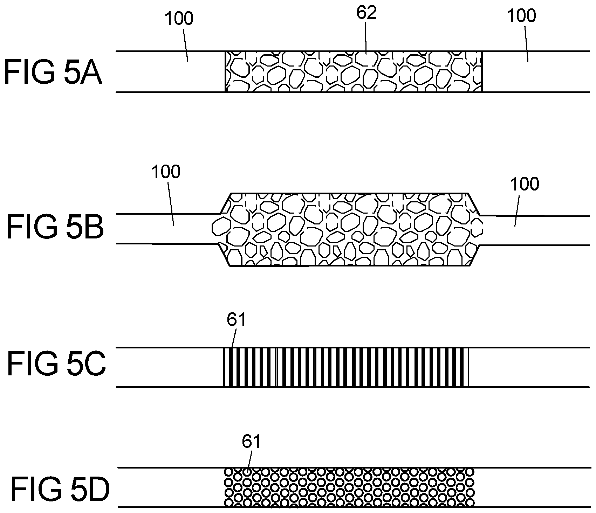

**Figur 5** stellt verschiedene Ausführungsformen des Fangelementes als integriertes Granulat z. B. Hydrogelpartikel oder funktionalisierte Silikapartikel dar. Figur 5 (A) stellt die Fixierung des Granulates an einer definierten Position eines Kanalabschnittes 100 durch ein Gitter 62 dar. Figur 5 (B) stellt eine Fixierung des Granulates durch eine Verengung des Kanalabschnitts 100 dar. In einer weiteren Ausführungsform kann das Fangelement in Form von Pfosten vorliegen, die dem Fluid eine möglichst große Oberfläche zur Verfügung stellen. Die Pfosten können als Hydrogelstrukturen ausgebildet sein oder als Silicapfosten vorliegen oder aus anderen funktionalisierten Strukturen (z. B. PMMA) gebildet werden. Figur 5 (C) zeigt eine Pfostenstruktur 61 in der Seitenansicht und Figur 5 (D) in der Draufsicht.

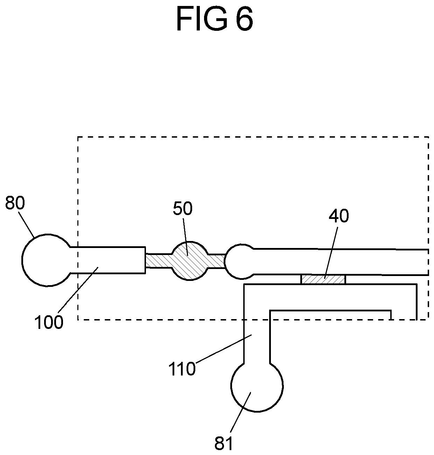

**Figur 6** stellt den prinzipiellen Aufbau der erfindungsgemäßen Vorrichtung dar. Die erfindungsgemäße Vorrichtung weist einen erster Kanalabschnitt 100 und einen zweiter Kanalabschnitt 110 auf, die peripher zueinander angeordnet sind. Der zweite Kanalabschnitt ist dabei über ein Öffnerelement 40 mit dem ersten Kanalabschnitt fluidisch verbindbar. Zudem ist im ersten Kanalabschnitt 100 dem Öffnerelement 40 ein Schließerelement 50 vorgelagert angeordnet. Der erste Kanalabschnitt 100 weist einen Aufnahmebereich 80 und der zweite Kanalabschnitt einen Aufnahmebereich 81 auf.

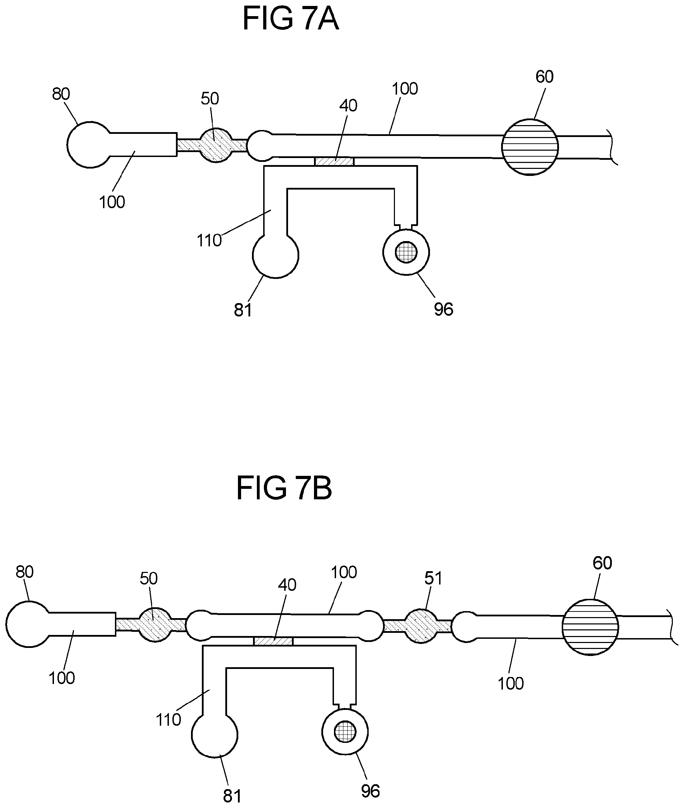

**Figur 7 (A) und (B)** stellen Ausführungsformen der vorliegenden Erfindung dar. Figur 7 (A) stellt einen ersten Kanalabschnitt 100 mit einem Aufnahmebereich 80 dar. In den Aufnahmebereich 80 wird eine fluidische Probe eingegeben, die durch den ersten Kanalabschnitt 100 bis zum Fangelement 60 und durch dieses hindurchströmt. Beim Durchströmen des ersten Kanalabschnittes 100 wird das Schließerelement 50 durch die fluidische Probe aktiviert, welches nach einer definierten Zeit den Kanalabschnitt 100 zum Aufnahmebereich 80 hin verschließt. Damit kann keine weitere Probe aus dem Aufnahmebereich 80 nachfließen, wodurch das Volumen der fluidischen Probe, welches durch das Fangelement 60 tritt, definiert wird.

In den Aufnahmebereich 81 eines zweiten Kanalabschnittes 110 wird ebenfalls eine Flüssigkeit eingebracht, die durch den zweiten Kanalabschnitt 110 bis zum Abluftventil 96 strömt. Durch das Abluftventil 96 kann die Luft aus dem Kanalabschnitt 110 entweichen, jedoch ist das Abluftventil 96 derart gestaltet, dass keine Flüssigkeit austritt. Durch die fluidische Probe im ersten Kanalabschnitt 100 und/oder durch die Flüssigkeit im zweiten Kanalabschnitt 110 wird das Öffnerelement 40 aktiviert. Die Barriere des Öffnerelementes 40 löst sich auf, bis ein fluidische Verbindung zwischen dem ersten Kanalabschnitt 100 und dem zweiten Kanalabschnitt 110 besteht, so dass die Flüssigkeit aus dem zweiten Kanalabschnitt 110 in den ersten Kanalabschnitt 100 strömen und in diesem dann durch das Fangelement 60 strömen kann.

Figur 7 (B) stellt einen ähnlichen Aufbau wie Figur 7 (A) dar, jedoch ist in dieser Anordnung ein weiteres Schließerelement 51 im ersten Kanalabschnitt 100 vorgesehen, welches hinter dem Öffnerelement 40 angeordnet ist. Das Schließerelement 51 wird durch die Flüssigkeit aus dem zweiten Kanalabschnitt 110 und/oder durch die zuvor hindurchgeströmte fluidische Probe aktiviert, wenn diese zum Fangelement 60 strömt. Nach einer definierten Zeit blockiert das Schließerelement 51 den ersten Kanalabschnitt 100 und es kann keine weitere Flüssigkeit vom zweiten Kanalabschnitt 110 zum Fangelement 60 nachfließen, wodurch das Volumen der Flüssigkeit aus dem zweiten Kanalabschnitt 110, welche durch das Fangelement 60 tritt, definiert wird.

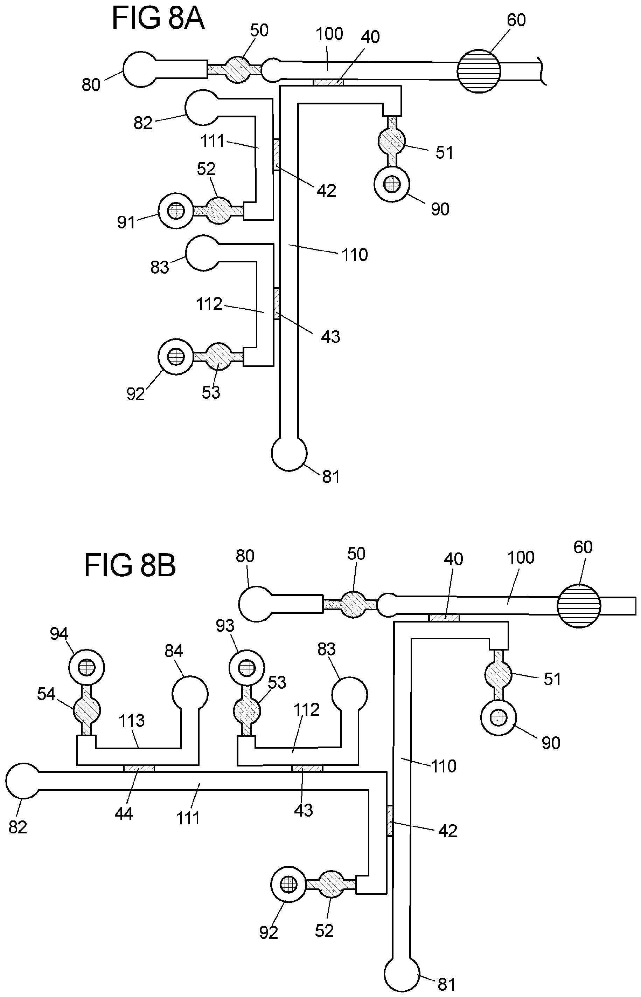

**Figur 8 (A)** stellt eine Ausführungsform der erfindungsgemäßen Vorrichtung dar. Der erste Kanalabschnitt 100 weist einen Aufnahmebereich 80 auf. In den Aufnahmebereich 80 wird eine fluidische Probe eingegeben, die durch den ersten Kanalabschnitt 100 bis zum Fangelement 60 und durch dieses hindurchströmt. Peripher zum ersten Kanal-schnitt 100 ist ein zweiter Kanalabschnitt 110 angeordnet. Der erste Kanalabschnitt 100 und der zweite Kanalabschnitt 110 sind durch ein erstes Öffnerelement 40 fluidisch verbindbar. Peripher zum zweiten Kanalabschnitt 110 sind zwei dritte Kanalabschnitte 111, 112 angeordnet, die durch jeweils ein Öffnerelement 42, 43 fluidisch mit dem zweiten Kanalabschnitt 110 verbunden werden können. Werden in die Aufnahmebereiche 80-83 des ersten und zweiten Kanalabschnitts und/oder dritten Kanalabschnitts fluidische Probe bzw. weitere Flüssigkeiten gegeben, wird der Auflösevorgang der jeweils an diese Kanalabschnitte angrenzenden Öffnerelemente gestartet. Nach einer definierten Zeit wird dadurch die fluidische Verbindung zwischen dem ersten Kanalabschnitt 100 und dem zweiten Kanalabschnitt 110 hergestellt und die Flüssigkeit aus dem zweiten Kanalabschnitt 110 strömt zum/durch das Fangelement 60. Nach einer definierten Zeit öffnen dann die Öffnerelemente 42, 43 und erlauben so einen sequentiellen Fluss von Prozessmedien zum bzw. durch das Fangelement 60.

**Figur 8 (B)** stellt eine weitere Ausführungsform der erfindungsgemäßen Vorrichtung dar. In dieser Ausführungsform weist die erfindungsgemäße Vorrichtung einen ersten Kanalabschnitt 100, einen zweiten Kanalabschnitt 110, einen dritten Kanalabschnitt 111 und zwei vierte Kanalabschnitte 112, 113. Dabei ist der zweite Kanalabschnitt 110 mit dem ersten Kanalabschnitt 100 über das Öffnerelement 40 fluidisch verbindbar. Der dritte Kanalabschnitt 111 wiederum ist über das Öffnerelement 42 mit dem zweiten Kanalabschnitt 110 fluidisch verbindbar. Die zwei vierten Kanalabschnitte 112 und 113 sind über die Öffnerelemente 43, 44 mit dem dritten Kanalabschnitt 111 fluidisch verbindbar. Weiterhin treffen alle bereits beschriebenen Merkmale auf diese Ausführungsform genauso zu.

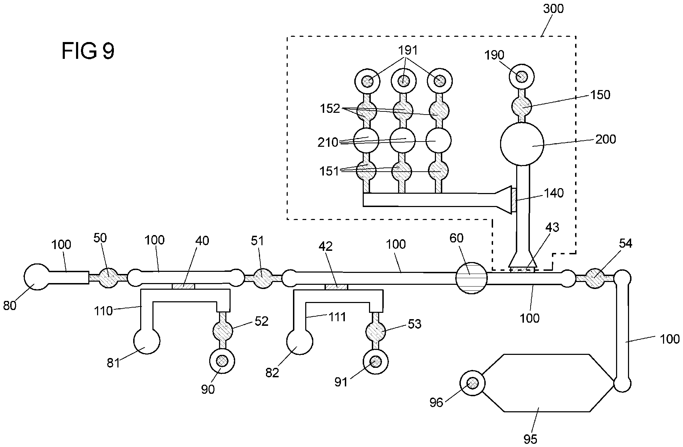

**Figur 9** stellt eine Ausführungsform der vorliegenden Erfindung dar, welches ein chemofluidisches System zur Vorbereitung einer Probe mit anschließendem Nachweis der Bestandteile darstellt. Das dargestellte Schaltbild einer LOC-Kanalstruktur ist für die Vorbereitung von z. B. biologischen Proben für eine nachfolgende Prozessierung, z. B. den Nachweis von Nukleinsäuren mittels isothermer Amplifikation, geeignet.

Über den Aufnahmebereich 80 kann eine fluidische Probe auf den ersten Kanalabschnitt 100 gegeben werden. Diese strömt durch den ersten Kanalabschnitt 100 über das Fangelement 60, welches eine permeable Membran sein kann, in die Abfallkammer 95. Durch ein in der Abfallkammer 95 platziertes Abluftventil 96 kann die in der Kanalstruktur vorhandene Luft entweichen, wobei keine Flüssigkeit austreten kann. Die fluidische Probe aktiviert auf ihrem Weg vom Aufnahmebereich 80 zur Abfallkammer 95 die Öffnerelemente 40, 42, 43 und die Schließerelemente 50, 51, 54. Das Schließerelement 50 schließt nach einer vordefinierten Zeit den ersten Kanalanschnitt zum Aufnahmebereich 80 hin und definiert somit das Probenvolumen, welches durch den ersten Kanalabschnitt 100 fließt.

Über den Aufnahmebereich 81 wird ein Puffer, z. B. ein Waschpuffer, auf den Kanalstrukturträger gegeben. Der Puffer fließt durch den zweiten Kanalabschnitt 110 zur permeablen Membran 90, welches Luft, aber keine Flüssigkeit aus der Kanalstruktur entweichen lässt. Die permeable Membran 90 ist Teil eines Abluftventils. An der permeablen Membran 90 kann weiterhin eine Auslassöffnung angeordnet sein, um eine Reinigung der Vorrichtung zu ermöglichen. Der Puffer aktiviert das Öffnerelement 40 zusätzlich von der Seite des zweiten Kanalabschnitts 110 und ebenso das Schließerelement 52. Das Schließerelement 52 unterstützt die permeable Membran 90 und stoppt den Flüssigkeitsstrom durch Verschließen des Strömungsweges nach einer vordefinierten Zeit. Nachdem das Schließerelement 50 den Strömungsweg verschlossen hat, öffnet das Öffnerelement 40 und lässt den Puffer vom zweiten Kanalabschnitt 110 in den ersten Kanalabschnitt 100 fließen. Der Puffer strömt über das Fangelement 60 und kann nicht immobilisierte Bestandteile von der permeablen Membran entfernen. Das Volumen des ersten Puffers wird über die Größe der Abfallkammer 95 sowie die Schließerelemente 51 und 54 definiert. Verschließt mindestens eines der Elemente den Strömungsweg, stoppt die Zugabe eines ersten Puffers.

Über den Aufnahmebereich 82 kann ein zweiter Puffer, z. B. ein Elutionspuffer, auf den Kanalstrukturträger aufgegeben werden. Dieser fließt in einem dritten Kanalabschnitt 111 zur permeablen Membran 91, welches Luft, aber keine Flüssigkeit aus der Kanalstruktur entweichen lässt. Die permeable Membran 91 ist Teil eines Abluftventils. An der permeablen Membran 91 kann weiterhin eine Auslassöffnung angeordnet sein, um eine Reinigung der Vorrichtung zu ermöglichen. Der zweite Puffer aktiviert zusätzlich das Öffnerelement 42 und weiterhin das Schließerelement 53. Das Schließerelement 53 ist Teil des Abluftventils und unterstützt die permeable Membran 91 und stoppt den Flüssigkeitsstrom durch Verschließen des Strömungsweges nach einer vordefinierten Zeit. Nachdem die Schließerelemente 51 und 54 den Strömungsweg verschlossen haben, öffnet das Öffnerelement 42 und lässt den zweiten Puffer vom weiteren zweiten Kanalabschnitt 111 in den ersten Kanalabschnitt 100 in Richtung der Kammer 200 fließen. Die Kammer 200 gehört zur Vorrichtung zur Probenanalytik 300, die durch das Öffnerelement 43 mit dem ersten Kanalabschnitt 100 verbunden ist.

Der zweite Puffer strömt ebenfalls über das Fangelement 60 und kann die dort immobilisierten Bestandteile der fluiden Probe ablösen. Die Kammer 200 verfügt über ein Schließerelement 150 und eine permeable Membran 190, die zusammen ein Abluftventil bilden. Das Schließerelement 150 wird durch den Fluidstrom aktiviert und verschließt sofort den Kanal, um - zusammen mit der permeablen Membran 190 - lediglich die Luft aus dem Strömungsweg entweichen zu lassen, nicht jedoch die Flüssigkeit. Die Flüssigkeit aktiviert auf ihrem Weg zu Kammer 200 das Öffnerelement 140. Nach einer vordefinierten Zeit öffnet dieses Öffnerelement 140 den Strömungsweg Richtung der Reaktionskammern 210, wobei jeweils ein Schließerelement 151 vor jeder Reaktionskammer und 152 nach jeder Reaktionskammer angeordnet ist, um das Volumen der Flüssigkeit in der Reaktionskammer 210 zu definieren und die Strömungswege sofort zu schließen. Die Schließerelemente 152 werden dabei von nachgelagerten permeablen Membranen 191 unterstützt und erlauben lediglich das Entweichen von Luft und nicht von Flüssigkeit.

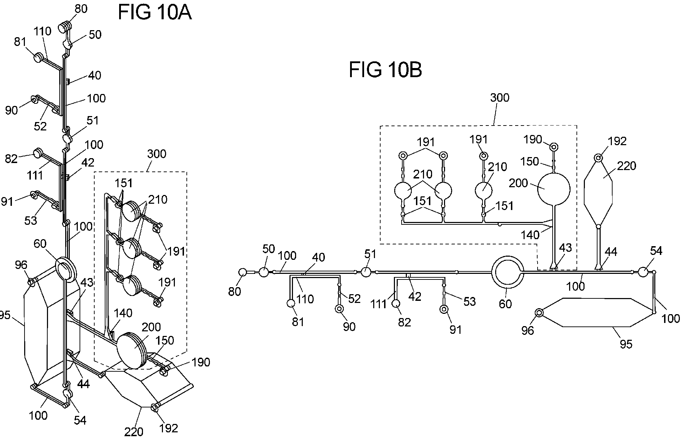

**Figur 10 (A) und (B)** stellen eine weitere Ausführungsform der vorliegenden Erfindung dar. Figur 10 (A) zeigt die Ausführungsform in einer 3D-Darstellung, während Figur 10 (B) die Ausführungsform in der Draufsicht zeigt. Der Aufbau der erfindungsgemäßen Vorrichtung entspricht grundlegend dem in der Figur 9 gezeigtem Aufbau. In dieser Ausführungsform sind zwei Abfallkammern 95, 220 nach dem Fangelement 60 angeordnet, wobei zweite Abfallkammer 220 über das Öffnerelement 44 mit dem ersten Kanalabschnitt verbunden ist. Dieses öffnet nach Befüllung der Abfallkammer 95 und Schließen des Schließerelementes 54. Die zweite Abfallkammer 220 weist ein nachgelagertes Abluftventil 192 auf. Die Vorrichtung für die Probenanalytik 300 ist über das Öffnerelemente 43 am ersten Kanalabschnitt 100 angeordnet. Die Vorrichtung ist in Strukturlayern aufgebaut.

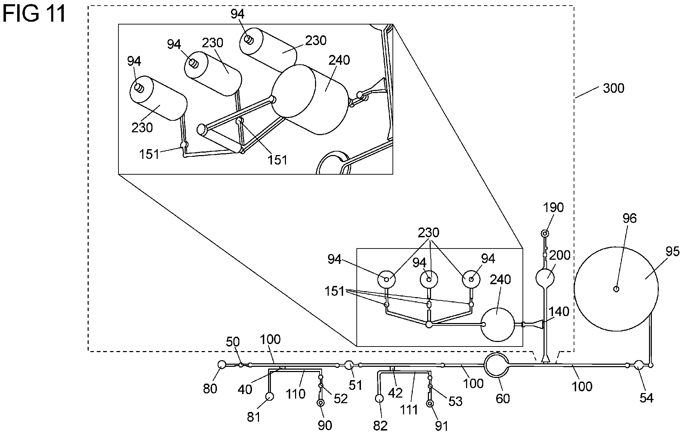

**Figur 11** stellt eine Ausführungsform der vorliegenden Erfindung mit einer Vorrichtung zur Probenanalytik 300 dar. Der Aufbau der erfindungsgemäßen Vorrichtung entspricht grundlegend dem in der Figur 9 gezeigtem Aufbau. In dieser Ausführungsform ist vor den drei Reaktionskammern 230 (Höhe: 4 mm, Durchmesser, 2,2 mm; Volumen jeweils 15 $\mu$l) eine Mischkammer 240 angeordnet. Diese erlaubt eine Durchmischung des Eluats vor dem Befüllen der Reaktionskammern 230, um einen möglichen Elutionsgradienten auszugleichen und die Befüllung jeder Reaktionskammer 230 mit der gleichen Konzentration des Analyten erlaubt. Jede Reaktionskammer 230 weist am Einlassbereich ein Schließerelement 151 auf sowie eine nachgelagerte permeable Membran.

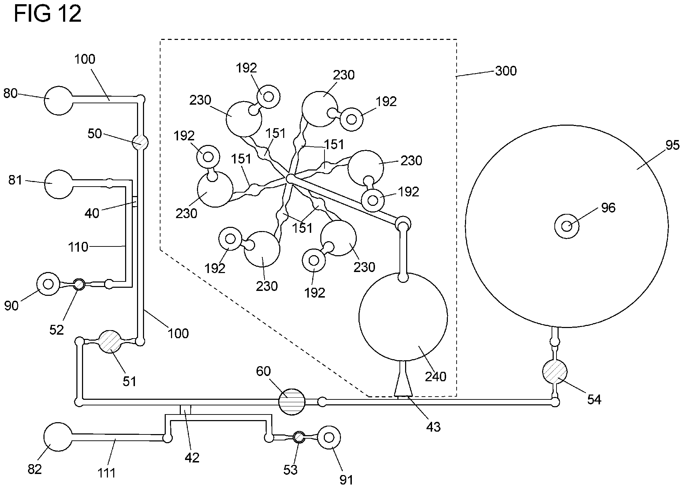

**Figur 12** stellt eine weitere Ausführungsform der vorliegenden Erfindung mit einer Probenanalytik 300 dar. Der Aufbau der erfindungsgemäßen Vorrichtung entspricht grundlegend dem in der Figur 11 gezeigtem Aufbau. In dieser Ausführungsform sind sechs Reaktionskammern (Höhe je 4 mm, Durchmesser je 1,75 mm, Volumen je 10 $\mu$l) derart angeordnet, dass eine gleichmäßige und parallele Befüllung dieser mit dem in der Mischkammer 240 (Höhe: 4 mm, Durchmesser: 4,4 mm, Volumen: 60 $\mu$l) durchmischten Eluats erfolgt. Jede Reaktionskammer 230 weist am Einlassbereich ein Schließerelement 151 auf sowie ein nachgelagertes Abluftventil 192.

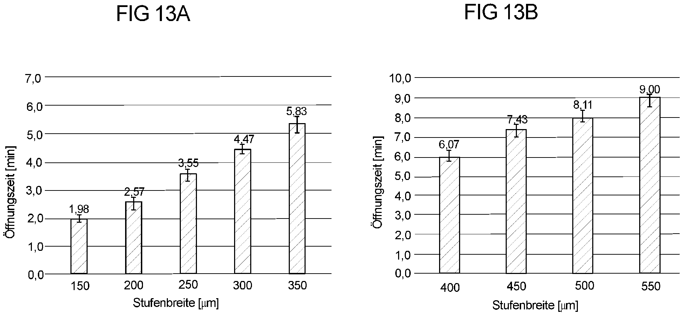

**Figur 13 (A) und (B)** stellen Öffnungszeiten für verschiedene Öffnerelemente 40 dar. Zur Simulation eines Extraktionsprozesses floss eine Lyse/Bindungspuffer für 2 min durch den ersten Kanalabschnitt und ein Waschpuffer für 10 s durch einen weiteren Kanalabschnitt. Der erste Kanalabschnitt und der weitere Kanalabschnitt waren durch ein Öffnerelement bestehend aus Polyethylenglykol (MW 1kDa und MW 1,5 kDa im Verhältnis 2:10) verbunden. Der Versuch wurde für verschiedenen Stufenbreiten des Öffnerelementes durchgeführt. In der Figur 13 (A) ist deutlich zu erkennen, wie die Öffnungszeit des Öffnerelementes von der Stufenbreite abhängt, bei ansonsten gleichen Versuchsparametern. Der Druck betrug 150 mbar bei einem Durchfluss von 100 $\mu$l/min, der Versuch wurde für jede Größe eines Öffnerelementes 3 mal durchgeführt.

**Figur 13 (A) und (B)** zeigt die Ergebnisse eines ähnlichen Versuchsaufbaus, nur das hier ein Elutionspuffer (LAMP-Puffer) 10 s durch den weiteren Kanalabschnitt geleitet wurde. Auch in dieser Versuchsanordnung ist eine Abhängigkeit der Öffnungszeit von der Stufenbreite deutlich zu erkennen.

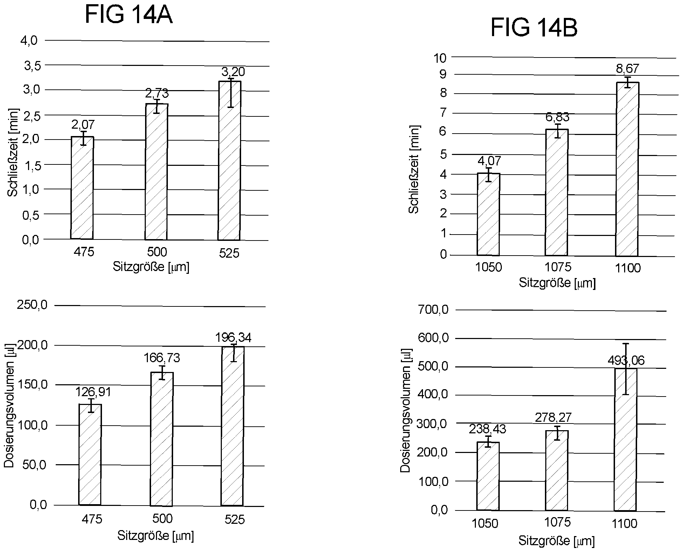

**Figur 14 (A) und (B)** stellen Schließzeiten für verschiedene Schließerelemente 50 dar. Die Schließerelemente basierten auf pDMAA (poly($N,N'$-dimethylacrylamid)) mit 10% BIS. Zur Simulation eines Extraktionsprotokolls ist ein Lyse/Bindungspuffer für 2 min durch ein Schließerelement geflossen und nach 4 min floss ein Waschpuffer hindurch, bis das Schließerelement vollständig geschlossen war. Der Druck betrug 150 mbar bei einem Durchfluss von 100 $\mu$l/min, der Versuch wurde für jede Größe eines Schließerelementes 4 mal durchgeführt.

**Figur 14 (A)** stellt die Schließzeiten und Dosierungsvolumen mit Lyse/Bindungspuffer für verschiedene Sitzgrößen dar. Das Schließerelement wies jeweils einen Durchmesser von 600 $\mu$m und eine Höhe von 300 $\mu$m im bei der Herstellung gequollenen Zustand auf.

**Figur 14 (B)** stellt die Schließzeiten und Dosierungsvolumen mit Lyse/Bindungspuffer für verschiedene Sitzgrößen dar. Das Schließerelement wies jeweils einen Durchmesser von 1200 $\mu$m und eine Höhe von 300 $\mu$m im bei der Herstellung gequollenen Zustand auf.

In beiden Versuchsanordnungen ist eine Abhängigkeit der Schließzeit von der Sitzgröße deutlich zu erkennen.

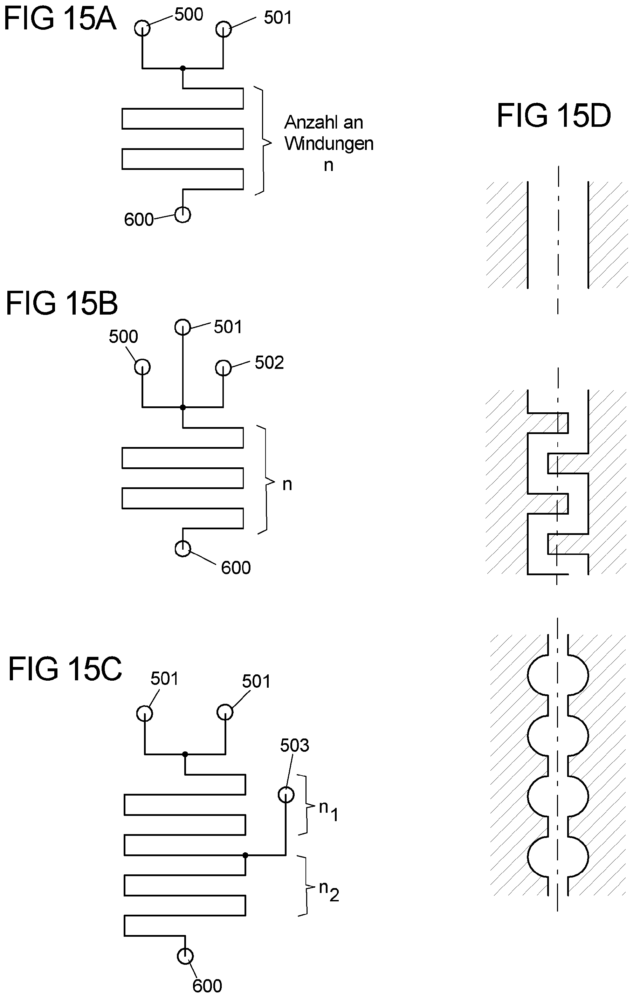

**Figur 15** (A), (B), (C) und (D) stellt vier verschiedene Ausführungsformen für den Aufnahmebereich dar. Figuren 11 (A) - (C) stellen jeweils verschiedene Ausführungsformen für Mischerstrukturen dar. Figur 15 (A) stellt eine Mischerstruktur für das Mischen von zwei Fluiden mit einer Meander-Struktur aus $n$- Windungen dar. Figur 15 (B) stellt eine Mischerstruktur für das gleichzeitige Mischen von drei Fluiden mit einer Meander-Struktur aus $n$-Windungen dar. Figur 15 (C) stellt eine Mischerstruktur für das Mischen von zunächst zwei Fluiden mit einer Meander-Struktur mit $n_1$-Windungen dar. Dann wird ein drittes Fluid zu den beiden bereits gemischten Fluiden zugegeben und entlang einer Meander-Struktur mit $n_2$-Windungen mit den beiden bereits gemischten Fluiden gemischt. Die Zugabe der Fluide erfolgt über die Inlets 500-503, das gemischte Fluid verlässt den Mischer über das Outlet 600. Figur 15 (D) stellt verschiedenen Kanalstrukturen dar. Gestrichelt ist dabei jeweils die Kanalmittellinie eingezeichnet.

## Ausführungsbeispiel 1

Es wurde ein Chip mit der erfindungsgemäßen Vorrichtung nach folgendem Protokoll hergestellt:

a. Aufbau: 7 verschiedene Layer

i. Layer 1 und Layer 3 bestehen aus hydrophilem Polyester (173 $\mu$m dick) mit beidseitigem Klebefilm (je 25 $\mu$m dick)

ii. Layer 2 sowie Layer 4 und 6 bestehen aus PMMA (250 $\mu$m dick)

iii. Layer 5 besteht aus PMMA (4 mm dick)

iv. Layer 7 besteht aus PMMA (250 $\mu$m dick mit Klebefilm (25 $\mu$m) auf einer Seite)

v. Die PTFE-Membran ist 100 $\mu$m dick mit 1,2 $\mu$m Poren

v. Cellulosemembran (Whatman Grade 1) mit Durchmesser von 1.2mm (nur einlagig möglich) bzw. 3mm (auch zweilagig möglich)

vi. Layer 0 besteht aus hydrophilem Polyester (wird auf einem Glasslide fixiert - Adhäsion durch Kapillarkräfte (Wassertropfen) oder mittels Klebefilm (25 $\mu$m))

b. Reinigung: bis auf Layer 0 werden alle Layer vor dem Zusammenbau 2x mit 0,1% Tween für je 15 Minuten im Ultraschallbad gewaschen, gefolgt von 2x doppelt destilliertes Wasser (15 Minuten), dann mit Stickstoff getrocknet und anschließend noch für 1 Stunde bei 55°C in den Trockenschrank gelegt. Zur Entfernung möglicher Rnasen erfolgt noch das Waschen mit einer Reinigungslösung für 10 Minuten gefolgt von drei Waschschritten mit DEPC-Wasser (mit Diethylpyrocarbonat behandeltes Wasser). Die Layer werden bei 55°C für 1 Stunde im Ofen getrocknet.

c. Fertigung des Chips: zunächst erfolgt die Polymerisation der Hydrogele zur Realisierung der Schließerelemente auf Layer 0 unter Schutzgasatmosphäre (Argon). Nach dem Waschen der Schließerelemente mit DEPC-Wasser und Trocknen über Nacht bei Raumtemperatur erfolgt das optische Alignment mit Layer 1. Beide Layer werden manuell laminiert. Anschließend erfolgt die Integration der löslichen Polymere zur Realisierung der Öffnerelemente. Nach optischem Alignment und Lamination werden die Stufen mit PEG befüllt und die Cellulose-Membran (Silica-Membran) integriert. Anschließend wird mittels optischem Alignment Layer 3 laminiert. Die Layer 4 bis 6 werden über ein Tool mit Pins zueinander ausgerichtet und in der Heißpresse bei 10 kN für 25 Minuten und bei 65°C verbunden. Anschließend erfolgt die Integration des LAMP-Mastermixes in den entsprechenden Kammern. Nach dem Trocknungsprozess des LAMP-Mastermixes werden die laminierten Layer 0-3 (Part 1) sowie die gepressten Layer 4-6 (Part 2) optisch zueinander ausgerichtet und kalt zusammengepresst (2,5 kN). Layer 7 enthält die PTFE-Membran, welche anschließend über die Pins mit dem Rest des Chips zueinander positioniert und kalt gepresst bei 2,5 kN wird.

Die erfindungsgemäße Vorrichtung auf dem Chip wies ein Design gemäß Figur 12 auf.

**Ausführungsbeispiel 2**

Der gemäß Ausführungsbeispiel 1 hergestellte Chip wurde wie folgt verwendet:

- Trocknung des LAMP-Mastermixes: 1.4mM dNTPs, 12 U Warmstart Bst2.0 (NEB), 5.7 U Warmstart RTx (NEB), 0.1% Tween, 1x Primermix (0.2$\mu$M F3/B3, 1.6$\mu$M FIB/BIP, 0.4$\mu$M FL/BL) und 3mM NaOH (bzgl Endvolumen). Gesamt-volumen=4.1$\mu$l; Reaktionsvolumen (nach Resuspension)=15$\mu$l
- LAMP-Chip mit LAMP-Mastermix wurde stehend für 2 Stunden bei 35°C getrocknet, damit Abluftkanäle nicht verstopfen
- Gleichzeitige Resuspension der 6 Reaktionskammern mit LAMP/Elutionspuffer (gespikt mit Covid-RNA BA5.0). Als Kontrolle wurde bei 3/6 Reaktionskammern Influenza A - Primer getrocknet
- Schließerelemente schließen innerhalb von einer Minute und verhindern die Vermischung der Mastermixe
- Gelbfärbung nur bei Covid19-Reaktionskammern.

**Ausführungsbeispiel 3**

Eine erfindungsgemäße Vorrichtung gemäß Figur 10 wurde wie folgt betrieben.

In den Aufnahmebereich 80 wurde ein Fluid eingegeben, welches aus einem Lyse/Bindungspuffer (200 $\mu$l) bestand. In den Aufnahmebereich 81 wurden 100 $\mu$l Waschpuffer aufweisend 50% Ethanol eingegeben und in den Aufnahmebereich 82 Wasser als Elutionspuffer. 3 Minuten nach Zugabe des Fluids schlossen die Schließerelemente 50 und 54. Durch das Verschließen des Schließerelements 50 wurde das Volumen des Lysats definiert. Zudem wurde die Abfallkammer 95 durch das Schließerelement 54 verschlossen. Nach 5 min öffnete das Öffnerelement 40 und das Öffnerelement 44. Der Waschpuffer konnte damit aus dem Kanalabschnitt 110 in den ersten Kanalabschnitt 100 strömen und über das Fangelement 60 in die Abfallkammer 220. Das Schließerelement 51 schloss sich nach 7 Minuten und die Öffnerelemente 42 und 43 öffneten nach 10 min. Hierdurch konnte der Elutionspuffer aus dem Kanalabschnitt 111 in den ersten Kanalabschnitt 100 strömen und über das Fangelement 60 und das Öffnerelement 43 in einen Bereich der Probenanalytik 300. Dabei wurde das Öffnerelement 140 aktiviert. Der erste Teil des Eluates wurde in der Abfallkammer 200 aufgefangen und schloss bei Kontakt mit dem Schließerelement 150 dieses sofort. 2 Minuten nach Aktivierung des Öffnerelementes 140 öffnete dieses den Strömungsweg in die Reaktionskammern 210.

**Ausführungsbeispiel 4**

Eine erfindungsgemäße Vorrichtung gemäß Figur 12 wurde wie folgt betrieben.

In den Aufnahmebereich 80 wurde ein Lysat eingegeben, welches aus einem VTM-Medium mit Covid-19 RNA (24 $\mu$l) in Lyse/Bindungspuffer (24 $\mu$l RLTplus Puffer + 67 $\mu$l Bindepuffer + 7 $\mu$l Carrier-RNA) bestand. In den Aufnahmebereich 81 wurden 100$\mu$l Waschpuffer (2,5 M NaCl, 10 mM Tris-HCl, pH8) eingegeben und in den Aufnahmebereich 82 Elutionspuffer (10 mM Ammoniumsulfat, 50 mM KCl, 8mM $MgSO_4$ in $ddH_2O$). In den Reaktionskammern 210 befand sich ein LAMP-Mastermix. 3 Minuten nach Zugabe des Lysats wurde der Zufluss gestoppt. Durch das Stoppen des Zuflusses des Lysats wurde das Volumen des Lysats auf 110 $\mu$l definiert. Nach 5 Minuten öffnete das Öffnerelement 40. Der Waschpuffer konnte damit aus dem Kanalabschnitt 110 in den ersten Kanalabschnitt 100 strömen und über das Fangelement 60 in die Abfallkammer 95. 7,7 Minuten nach Zugabe des Lysats schloss das Schließerelement 54. Zudem wurde die Abfallkammer 95 durch das Schließerelement 54 verschlossen.

Das Schließerelement 51 schloss sich nach 7,7 Minuten und die Öffnerelemente 42 und 43 öffneten sich nach 31 Minuten. Hierdurch konnte der Elutionspuffer aus dem Kanalabschnitt 111 in den ersten Kanalabschnitt 100 strömen und über das Fangelement 60 und das Öffnerelement 43 in einen Bereich der Probenanalytik 300. Das Eluat sammelte sich zunächst in der Mischkammer 240 und strömte nach dem Befüllen der Mischkammer in die Reaktionskammern 230 und aktivierte hierbei die Schließerelemente 151, die innerhalb von 1-2 Minuten schlossen. Die Isolationseffizienz für das E-Gene und das S-Gen der Covid-19 RNA lagen bei 98,1 bzw. 88,9%.

**Bezugszeichenliste**

| | |
|---|---|
| 100, 110, 111, 112, 113 | Kanalabschnitt |
| 10, 11, 14 | Strukturträger |
| 12, 13 | Abdeckung |
| 40, 42, 43, 44 | Öffnerelement |
| 40a | kanalförmiger Teil |
| 40b, 40c | Verbindungselement |
| 41 | Verbindung |
| 50-54 | Schließerelement |
| 60 | Fangelement |
| 61 | Gitter |
| 62 | Pfostenstruktur |
| 80-84 | Aufnahmebereich |
| 90-94, 190, 191 | permeable Membran |
| 95 | Abfallkammer |
| 96 | Abluftventil |
| 140 | Öffnerelement |
| 150,151, 152 | Schließerelement |
| 192 | Abluftventil |
| 200 | (Abfall)Kammer |
| 210 | Reaktionskammer |
| 220 | Abfallkammer |
| 230 | Reaktionskammer |
| 240 | Mischkammer |
| 300 | Probenanalytik |
| 500-503 | Inlet |
| 600 | Outlet |
| a | Stufenbreite |

**Patentansprüche**

1. Fluidische Vorrichtung, aufweisend einen ersten Kanalabschnitt (100) und n zweite Kanalabschnitte (110-113), wobei $n \in \mathbb{N}$ und $n \geq 1$, mindestens ein Öffnerelement (40-44) und mindestens ein Schließerelement (50-54) **dadurch gekennzeichnet, dass**

   • jeder der n zweiten Kanalabschnitte (110-113) durch ein Öffnerelement (40-44) angrenzend an den ersten Kanalabschnitt (100) angeordnet ist;
   • jedes Öffnerelement (40-44) dazu eingerichtet ist, eine fluidische Verbindung von dem jeweiligen zweiten Kanalabschnitt (110-113) zum ersten Kanalabschnitt (100) herzustellen;
   • der erste (100) und die n zweiten Kanalabschnitte (110-113) jeweils einen Aufnahmebereich (80-84) aufweisen,
   • im ersten Kanalabschnitt (100) in Strömungsrichtung eines Fluids mindestens einem der Öffnerelemente (40-44) ein Schließerelement (50-54) vorgelagert angeordnet ist.

2. Fluidische Vorrichtung gemäß Anspruch 1, **dadurch gekennzeichnet, dass** $n \geq 2$ und jeder der n zweiten Kanalabschnitte (110-113) beabstandet zueinander in Reihe am ersten Kanalabschnitt (100) angeordnet ist.

3. Fluidische Vorrichtung gemäß einem der vorangegangenen Ansprüche, **dadurch gekennzeichnet, dass** die fluidische Vorrichtung weiterhin m dritte Kanalabschnitte (110-113) aufweist, wobei $m \in \mathbb{N}$ und $m \geq 1$, und jeder der m dritten Kanalabschnitte (110-113) jeweils durch ein Öffnerelement (40-44) angrenzend an zumindest einen der n zweiten Kanalabschnitte (110-113) angeordnet ist und das jeweilige Öffnerelement (40-44) dazu eingerichtet ist, eine fluidische Verbindung von einem dritten Kanalabschnitt (110-113) zu einem zweiten Kanalabschnitt (110-113) herzustellen.

4. Fluidische Vorrichtung gemäß Anspruch 3, **dadurch gekennzeichnet, dass** die fluidische Vorrichtung weiterhin f k-te Kanalabschnitte (110-113) aufweist, wobei $f \in \mathbb{N}$ und $f \geq 1$ sowie $k \in \mathbb{N}$ und $k \geq 4$ jeder der f k-ten Kanalabschnitte (110-113) jeweils durch ein Öffnerelement (40-44) angrenzend an zumindest einen der (k-1)-ten Kanalabschnitte (110-113) angeordnet ist und das jeweilige Öffnerelement (40-44) dazu eingerichtet ist, eine fluidische Verbindung von einem k-ten Kanalabschnitt (110-113) zu einem (k-1)-ten Kanalabschnitt (110-113) herzustellen.

5. Fluidische Vorrichtung gemäß einem der vorangegangenen Ansprüche, **dadurch gekennzeichnet, dass** der erste Kanalabschnitt (100) weiterhin ein Fangelement (60) aufweist, welches in Strömungsrichtung eines Fluids nach dem letzten Öffnerelement (40-44) welches einen der n zweiten Kanalabschnitte (110-113) mit dem ersten Kanalabschnitt (100) verbindet, im ersten Kanalabschnitt (100) angeordnet ist.

6. Fluidische Vorrichtung gemäß Anspruch 5, **dadurch gekennzeichnet, dass** das Fangelement (60) ein permeables Element ist.

7. Fluidische Vorrichtung gemäß einem der vorangegangenen Ansprüche, **dadurch gekennzeichnet, dass** die fluidische Vorrichtung weiterhin eine oder mehrere Druck- und/oder Flussquellen aufweist.

8. Fluidische Vorrichtung gemäß einem der vorangegangenen Ansprüche, **dadurch gekennzeichnet, dass** die Öffnerelemente (40-44) lösliche Barrieren sind.

9. Fluidische Vorrichtung gemäß einem der vorangegangenen Ansprüche, **dadurch gekennzeichnet, dass** die Schließerelemente (50-54) Quellmittelbarrieren sind.

10. Fluidische Vorrichtung gemäß Anspruch 5, **dadurch gekennzeichnet, dass** die fluidische Vorrichtung weiterhin eine Vorrichtung zur Probenanalytik (300) aufweist, welche in Strömungsrichtung eines Fluids nach dem Fangbereich (60) des ersten Kanalabschnitts (100) angeordnet ist, und mit dem ersten Kanalabschnitt (100), vorzugsweise durch mindestens ein Öffnerelement (40-44), fluidisch verbunden ist.

11. Fluidische Vorrichtung gemäß Anspruch 10, **dadurch gekennzeichnet, dass** die Vorrichtung zur Probenanalytik (300) eine Vorrichtung zum Nachweis/zur Analyse von Analyten ist,

wobei die Vorrichtung zur Probenanalytik (300) j Reaktionskammern (230) aufweist, wobei $j \in \mathbb{N}$ und $j \geq 1$;
wobei jede Reaktionskammer (230) einen Einlass und einen Auslass aufweist; und
wobei in Strömungsrichtung eines Fluids vor dem Einlass jeder Reaktionskammer (230) ein Schließerelement (50-54) angeordnet ist und am Auslass jeder Reaktionskammer (230) ein Abluftventil (192) und/oder Schließerelement (50-54) angeordnet ist.

12. Fluidische Vorrichtung gemäß einem der Ansprüche 10 oder 11, **dadurch gekennzeichnet, dass** die Vorrichtung zur Probenanalytik (300) weiterhin eine Mischkammer (240) aufweist, die dazu eingerichtet ist einen Konzentrationsausgleich in einem Fluid zu bewirken.

13. Verfahren zur sequentiellen Bereitstellung von definierten Fluidvolumina mit einer fluidischen Vorrichtung aufweisend einen ersten Kanalabschnitt (100) und mindestens einen zweiten Kanalabschnitt (110-113), mindestens ein Öffnerelement (40-44) und mindestens ein Schließerelement (50-54)
wobei

• der mindestens eine zweite Kanalabschnitt (110-113) durch ein Öffnerelement (40-44) angrenzend an den ersten Kanalabschnitt (100) angeordnet ist und das Öffnerelement (40-44) dazu eingerichtet ist, eine fluidische Verbindung von dem mindestens einen zweiten Kanalabschnitt (110-113) zum ersten Kanalabschnitt (100) herzustellen;
• der erste (100) und der mindestens eine zweite Kanalabschnitt (110-113) jeweils einen Aufnahmebereich (80-84) aufweisen,
• im ersten Kanalabschnitt (100) in Strömungsrichtung eines Fluids dem Öffnerelement (40-44) ein Schließerelement (50-54) vorgelagert angeordnet ist;

**umfassend die Schritte**

a) Bereitstellen eines ersten Fluids im Aufnahmebereich (80-84) des ersten Kanalabschnitts (100), wodurch eine Strömungsrichtung des ersten Fluids in dem ersten Kanalabschnitt (100) ausgebildet wird;
b) Bereitstellen eines zweiten Fluids in einem Aufnahmebereich (80-84) eines mindestens einen zweiten Kanalabschnitts (110-113), wodurch eine Strömungsrichtung des zweiten Fluids in dem mindestens einen zweiten Kanalabschnitt (110-113) ausgebildet wird;
c) Aktivieren des Schließerelementes (50-54) durch das erste Fluid im ersten Kanalabschnitt (100) und Strömen des ersten Fluids durch den ersten Kanalabschnitts (100) bis das Schließerelement (50-54) geschlossen ist;
d) Aktivieren des mindestens einen Öffnerelementes (40-44) durch das erste Fluid im ersten Kanalabschnitt (100) und/oder aktivieren des mindestens einen Öffnerelementes (40-44) durch das zweite Fluid im mindestens einen zweiten Kanalabschnitt (110-113) und Öffnen einer fluidischen Verbindung zwischen dem ersten (100) und dem mindestens einen zweiten Kanalabschnitt (110-113);
e) Einströmen des zweiten Fluids aus dem mindestens einen zweiten Kanalabschnitt (110-113) in den ersten Kanalabschnitt (100) und Durchströmen des ersten Kanalabschnitt (100) in Strömungsrichtung des ersten Fluids.

14. Verfahren zur sequentiellen Bereitstellung von definierten Fluidvolumina gemäß Anspruch 13, **dadurch gekennzeichnet, dass** die fluidische Vorrichtung weiterhin ein Fangelement (60) im ersten Kanalabschnitt (100) nach dem Öffnerelement (40-44) aufweist und die Fluide diese durchströmen.

15. Verfahren zur sequentiellen Bereitstellung von definierten Fluidvolumina gemäß Anspruch 13 oder 14, **dadurch gekennzeichnet, dass** die fluidische Vorrichtung weiterhin eine Vorrichtung zur Probenanalytik (300) aufweist, welche durch ein Öffnerelement (40-44) am ersten Kanalabschnitt (100) angeordnet ist, wobei das Öffnerelement (40-44) dazu eingerichtet ist eine fluidische Verbindung vom ersten Kanalabschnitt (100) zu der Vorrichtung zur Probenanalytik (300) herzustellen;

wobei die Vorrichtung zur Probenanalytik (300) in Strömungsrichtung eines Fluids hinter dem Öffnerelement (40-44) angeordnet ist, welches den zweiten (110-113) mit dem ersten Kanalabschnitt (100) verbindet oder hinter dem Fangelement (60) im ersten Kanalabschnitt (100);
und wobei ein Fluidstrom im ersten Kanalabschnitt (100) das Öffnerelement (40-44) aktiviert, welches die Vorrichtung zur Probenanalytik (300) mit dem ersten Kanalabschnitt (100) fluidisch verbindet.

## FIG 1A

## FIG 1B

# FIG 2

# FIG 3A

# FIG 3B

# FIG 4A

# FIG 4B

FIG 5A

100    62    100

FIG 5B

100    100

FIG 5C

61

FIG 5D

61

# FIG 6

# FIG 7A

# FIG 7B

## FIG 8A

## FIG 8B

FIG 9

FIG 10A

FIG 10B

FIG 11

## FIG 12

FIG 13A

FIG 13B

## FIG 14A

Schließzeit [min] / Sitzgröße [µm]

475: 2,07
500: 2,73
525: 3,20

Dosierungsvolumen [µl] / Sitzgröße [µm]

475: 126,91
500: 166,73
525: 196,34

## FIG 14B

Schließzeit [min] / Sitzgröße [µm]

1050: 4,07
1075: 6,83
1100: 8,67

Dosierungsvolumen [µl] / Sitzgröße [µm]

1050: 238,43
1075: 278,27
1100: 493,06

FIG 15A

500 501

600

Anzahl an
Windungen
n

FIG 15B

500 501

502

n

600

FIG 15C

501 501

503

n$_1$

n$_2$

600

FIG 15D

Europäisches Patentamt
European Patent Office
Office européen des brevets

# EUROPÄISCHER RECHERCHENBERICHT

**Nummer der Anmeldung**

EP 24 20 8349

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2015/044688 A1 (RICHTER ANDREAS [DE] ET AL) 12. Februar 2015 (2015-02-12) | 1-12 | INV. B01L3/00 |
| A | * Zusammenfassung * * Absätze [0064], [0065]; Abbildungen 1,2a,2b * | 13-15 | B01L7/00 |
| | - - - - - | | |
| A | US 2011/126913 A1 (BANERJEE DEBJYOTI [US] ET AL) 2. Juni 2011 (2011-06-02) * Zusammenfassung * * Absätze [0118] - [0120]; Abbildungen 5A-E * | 13-15 | |
| | - - - - - | | |
| A | DE 10 2010 015161 A1 (UNIV DRESDEN TECH [DE]) 20. Oktober 2011 (2011-10-20) * das ganze Dokument * | 1-15 | |
| | - - - - - | | |
| A | US 2010/240022 A1 (MCNEELY MICHAEL R [US]) 23. September 2010 (2010-09-23) * das ganze Dokument * | 1-15 | |
| | - - - - - | | |

**RECHERCHIERTE SACHGEBIETE (IPC)**

B01L

Der vorliegende Recherchenbericht wurde für alle Patentansprüche erstellt

| Recherchenort | Abschlußdatum der Recherche | Prüfer |
|---|---|---|
| Den Haag | 20. Februar 2025 | Sinn, Cornelia |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
   
& : Mitglied der gleichen Patentfamilie, übereinstimmendes
   Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 24 20 8349

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-02-2025

| Im Recherchenbericht angeführtes Patentdokument | Datum der Veröffentlichung | Mitglied(er) der Patentfamilie | Datum der Veröffentlichung |
|---|---|---|---|
| US 2015044688 A1 | 12-02-2015 | DE 102012206042 A1 | 31-10-2013 |
| | | EP 2836302 A1 | 18-02-2015 |
| | | US 2015044688 A1 | 12-02-2015 |
| | | WO 2013153181 A1 | 17-10-2013 |
| US 2011126913 A1 | 02-06-2011 | EP 1824600 A2 | 29-08-2007 |
| | | EP 1824601 A2 | 29-08-2007 |
| | | EP 1828746 A2 | 05-09-2007 |
| | | US 8312890 B1 | 20-11-2012 |
| | | US 2006090800 A1 | 04-05-2006 |
| | | US 2006093526 A1 | 04-05-2006 |
| | | US 2006093528 A1 | 04-05-2006 |
| | | US 2010133104 A1 | 03-06-2010 |
| | | US 2010136701 A1 | 03-06-2010 |
| | | US 2011114206 A1 | 19-05-2011 |
| | | US 2011126913 A1 | 02-06-2011 |
| | | US 2012214156 A1 | 23-08-2012 |
| | | WO 2006044841 A2 | 27-04-2006 |
| | | WO 2006044843 A2 | 27-04-2006 |
| | | WO 2006044896 A2 | 27-04-2006 |
| DE 102010015161 A1 | 20-10-2011 | DE 102010015161 A1 | 20-10-2011 |
| | | WO 2011127908 A1 | 20-10-2011 |
| US 2010240022 A1 | 23-09-2010 | US 2010240022 A1 | 23-09-2010 |
| | | WO 2008002483 A2 | 03-01-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 10814321 B
- US 20200215544 A
- US 10947528 B
- US 10843188 B
- US 11142757 B
- US 10940473 B
- EP 2836302 A
- DE 112007003160
- DE 102010015161